(19) Europäisches Patentamt
European Patent Office
Office européen des brevets

(11) EP 4 635 945 A1

(12) EUROPEAN PATENT APPLICATION
published in accordance with Art. 153(4) EPC

(43) Date of publication:
22.10.2025 Bulletin 2025/43

(21) Application number: 23902608.1

(22) Date of filing: 08.12.2023

(51) International Patent Classification (IPC):
*C07D 263/32* (2006.01) *A61P 37/02* (2006.01)
*A61P 37/04* (2006.01) *A61P 37/06* (2006.01)
*A61P 19/02* (2006.01) *A61K 31/421* (2006.01)

(52) Cooperative Patent Classification (CPC):
**A61K 31/421; A61P 19/02; A61P 37/02; A61P 37/04; A61P 37/06; C07D 263/32**

(86) International application number:
**PCT/CN2023/137500**

(87) International publication number:
**WO 2024/125412 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.12.2022 CN 202211596990**

(71) Applicants:
- **Jiankuan (Suzhou) Biotechnology Co., Ltd**
  **Suzhou, Jiangsu 215000 (CN)**
- **Institute of Materia Medica,**
  **Chinese Academy of Medical Sciences**
  **Beijing 100050 (CN)**

(72) Inventors:
- **WANG, Xiaojian**
  **Suzhou, Jiangsu 215000 (CN)**
- **CHEN, Xiaoguang**
  **Suzhou, Jiangsu 215000 (CN)**
- **ZHAO, Yanshi**
  **Suzhou, Jiangsu 215000 (CN)**
- **WU, Ping**
  **Suzhou, Jiangsu 215000 (CN)**
- **JIN, Jing**
  **Suzhou, Jiangsu 215000 (CN)**
- **LI, Yan**
  **Suzhou, Jiangsu 215000 (CN)**
- **SHENG, Li**
  **Suzhou, Jiangsu 215000 (CN)**
- **CHEN, Xin**
  **Suzhou, Jiangsu 215000 (CN)**

(74) Representative: **Regimbeau**
**20, rue de Chazelles**
**75847 Paris Cedex 17 (FR)**

# (54) CRYSTAL FORM OF PROXIMOD, PREPARATION METHOD THEREFOR, AND USE THEREOF

(57) The present application provides a crystal form of proximod, a preparation method therefor, and use thereof. The crystal form relates to a crystal form A and a crystal form B. The crystal form A and the crystal form B can stably exist, have low hygroscopicity, high solubility, good mechanical stability, low fluidity, low adhesiveness, and good compressibility, and have pharmaceutical industrialization prospects.

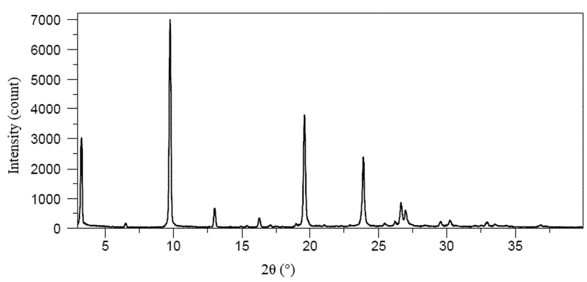


FIG. 1

EP 4 635 945 A1

## Description

### TECHNICAL FIELD

The present application belongs to the field of medicines and, in particular, relates to crystal forms of proximod, a preparation method therefor, and use thereof.

### BACKGROUND

Proximod (2-amino-2-{2-(4'-(2-propyloxazol-4-yl)-[1,1'-biphenyl]-4-yl)ethyl}-1,3-propanediol hydrochloride, H001) is an S1P1 receptor agonist and belongs to prodrugs. Proximod is phosphorylated into H001-P *in vivo* to agonize the S1P1 receptor and can be used for preparing drugs for treating autoimmune diseases.

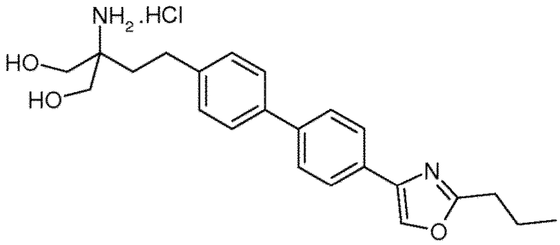

Proximod (H001)

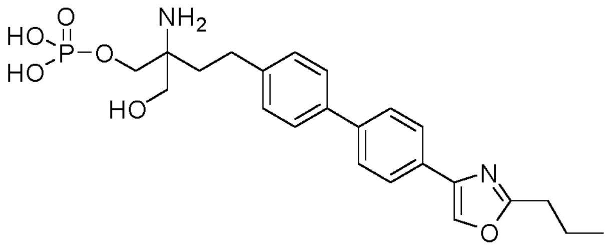

H001-P

Although the existing art has disclosed the methods for preparing proximod, the crystal forms of proximod are not disclosed yet. If the primary drug ingredient used in the preparation process of a pharmaceutical preparation is a mixed crystal, the dissolution performance and drug efficacy of the resulting preparation product may be affected, so the research on the crystal forms of proximod is of great significance to the subsequent preparation research and development.

### SUMMARY

The present application provides crystal forms of proximod, a preparation method therefor, and use thereof.

In a first aspect, the present application provides a crystal form A of proximod. An X-ray powder diffraction (XRPD) pattern of the crystal form A has diffraction peaks at the following diffraction angles (2θ): 3.2° ± 0.2°, 9.7° ± 0.2°, 12.9° ± 0.2°, 16.2° ± 0.2°, 19.5° ± 0.2°, 23.8° ± 0.2°, 26.5° ± 0.2°, and 26.8° ± 0.2°.

In the present application, the XRPD pattern of the crystal form A is shown in FIG. 1, and the XRPD parameters of the crystal form A are shown in the following table:

| 2θ (°) | d (Å) | Height (counts) | $I/I_0$ (%) | FWHM (°) |
|---|---|---|---|---|
| 3.208 | 27.5164 | 9163 | 39.5 | 0.096 |
| 5.166 | 17.0908 | 22 | 0.1 | 0.072 |
| 6.080 | 14.5260 | 28 | 0.1 | 0.072 |
| 6.426 | 13.7427 | 598 | 2.6 | 0.120 |
| 6.835 | 12.9221 | 30 | 0.1 | 0.072 |
| 9.688 | 9.1222 | 23173 | 100.0 | 0.096 |
| 12.033 | 7.3489 | 7 | 0.0 | 0.192 |
| 12.946 | 6.8329 | 2002 | 8.6 | 0.120 |
| 16.195 | 5.4686 | 739 | 3.2 | 0.072 |
| 17.037 | 5.2002 | 55 | 0.2 | 0.144 |
| 18.512 | 4.7891 | 38 | 0.2 | 0.072 |
| 19.483 | 4.5526 | 11154 | 48.1 | 0.120 |
| 20.927 | 4.2416 | 127 | 0.6 | 0.096 |

(continued)

| 2θ (°) | d (Å) | Height (counts) | $I/I_0$ (%) | FWHM (°) |
|---|---|---|---|---|
| 23.778 | 3.7390 | 3306 | 14.3 | 0.120 |
| 25.375 | 3.5072 | 129 | 0.6 | 0.216 |
| 26.116 | 3.4094 | 439 | 1.9 | 0.120 |
| 26.500 | 3.3608 | 1305 | 5.6 | 0.072 |
| 26.845 | 3.3184 | 1170 | 5.1 | 0.096 |
| 27.619 | 3.2272 | 16 | 0.1 | 0.072 |
| 29.402 | 3.0354 | 402 | 1.7 | 0.120 |
| 30.085 | 2.9680 | 681 | 2.9 | 0.096 |
| 32.774 | 2.7303 | 472 | 2.0 | 0.096 |
| 33.335 | 2.6857 | 245 | 1.1 | 0.120 |
| 33.569 | 2.6675 | 25 | 0.1 | 0.072 |
| 33.832 | 2.6474 | 9 | 0.0 | 0.144 |
| 34.226 | 2.6178 | 127 | 0.6 | 0.120 |
| 36.731 | 2.4448 | 240 | 1.0 | 0.120 |
| 37.132 | 2.4193 | 94 | 0.4 | 0.072 |
| 38.279 | 2.3494 | 9 | 0.0 | 0.144 |

In a second aspect, the present application provides a method for preparing the crystal form A of proximod described in the first aspect. The preparation method includes the following steps: adding a crude sample of proximod to a mixed solvent of tetrahydrofuran and water, keep a pH of the system at 6-6.5, heating to dissolve the crude sample to a clear solution, adding the clear solution to methyl *tert*-butyl ether and stirring, precipitating a solid, separating the solid and drying under reduced pressure to obtain the crystal form A.

Preferably, a volume ratio of tetrahydrofuran to water in the mixed solvent is 9: 1.

Preferably, the heating is heating to reflux.

In the present application, the clear solution is added to methyl *tert*-butyl ether dropwise.

Preferably, the solid is dried under reduced pressure at a temperature of 40 °C to 50 °C, for example, 40 °C, 42 °C, 45 °C, 48 °C or 50 °C.

In the present application, during the preparation of the crystal form A, the pH of the system is kept at 6-6.5, for example, 6, 6.1, 6.2, 6.3, 6.4 or 6.5, etc. The means for achieving the pH of the system of 6 to 6.5 may be the means commonly used in the art for adjusting the pH value. For example, the pH value may be adjusted by adding an acid or alkali or by adding a buffer.

In the present application, the crystal form A may be obtained by keeping the pH of the system at 6-6.5 during the preparation of the crystal form. If the pH of the system changes, for example, if the pH of the system is less than 6, a mixed crystal is formed, and if the pH of the system is greater than 6.5, no crystal can be formed.

In a third aspect, the present application provides a crystal form B of proximod. An X-ray powder diffraction pattern of the crystal form B has diffraction peaks at the following diffraction angles (2θ): 4.5° ± 0.2°, 9.0° ± 0.2°, 13.6° ± 0.2°, 18.1° ± 0.2°, 19.2° ± 0.2°, 21.8° ± 0.2°, 24.5° ± 0.2°, 27.5° ± 0.2°, and 27.8° ± 0.2°.

In the present application, the XRPD pattern of the crystal form B is shown in FIG. 6, and the XRPD parameters are shown in the following table:

| 2θ (°) | d (Å) | Height (counts) | $I/I_0$ (%) | FWHM (°) |
|---|---|---|---|---|
| 4.495 | 19.6424 | 1074 | 54.9 | 0.096 |
| 7.794 | 11.3345 | 47 | 2.4 | 0.072 |
| 8.670 | 10.1906 | 40 | 2.0 | 0.072 |
| 9.009 | 9.8078 | 1001 | 51.2 | 0.120 |
| 9.350 | 9.4510 | 32 | 1.6 | 0.072 |

(continued)

| 2θ (°) | d (Å) | Height (counts) | $I/I_0$ (%) | FWHM (°) |
|---|---|---|---|---|
| 9.708 | 9.1035 | 308 | 15.8 | 0.096 |
| 10.215 | 8.6531 | 20 | 1.0 | 0.072 |
| 10.775 | 8.2040 | 153 | 7.8 | 0.120 |
| 11.206 | 7.8894 | 20 | 1.0 | 0.144 |
| 12.018 | 7.3584 | 242 | 12.4 | 0.144 |
| 13.575 | 6.5178 | 912 | 46.7 | 0.096 |
| 14.770 | 5.9930 | 10 | 0.5 | 0.120 |
| 15.388 | 5.7536 | 75 | 3.8 | 0.072 |
| 15.720 | 5.6329 | 19 | 1.0 | 0.072 |
| 16.138 | 5.4878 | 230 | 11.8 | 0.096 |
| 16.542 | 5.3547 | 16 | 0.8 | 0.072 |
| 17.528 | 5.0556 | 274 | 14.0 | 0.120 |
| 17.821 | 4.9732 | 167 | 8.6 | 0.120 |
| 18.138 | 4.8870 | 448 | 22.9 | 0.096 |
| 18.532 | 4.7838 | 320 | 16.4 | 0.120 |
| 19.258 | 4.6052 | 1279 | 65.4 | 0.096 |
| 19.538 | 4.5398 | 354 | 18.1 | 0.096 |
| 20.553 | 4.3178 | 216 | 11.0 | 0.096 |
| 20.994 | 4.2281 | 92 | 4.7 | 0.096 |
| 21.541 | 4.1221 | 390 | 20.0 | 0.072 |
| 21.764 | 4.0803 | 1956 | 100.0 | 0.120 |
| 22.336 | 3.9771 | 290 | 14.8 | 0.096 |
| 22.733 | 3.9085 | 209 | 10.7 | 0.096 |
| 24.239 | 3.6690 | 294 | 15.0 | 0.144 |
| 24.531 | 3.6259 | 875 | 44.8 | 0.096 |
| 25.527 | 3.4867 | 205 | 10.5 | 0.120 |
| 25.733 | 3.4592 | 172 | 8.8 | 0.096 |
| 26.464 | 3.3653 | 83 | 4.2 | 0.120 |
| 27.469 | 3.2444 | 468 | 23.9 | 0.096 |
| 27.755 | 3.2117 | 670 | 34.3 | 0.072 |
| 30.364 | 2.9414 | 437 | 22.4 | 0.120 |
| 31.098 | 2.8735 | 231 | 11.8 | 0.072 |
| 31.413 | 2.8455 | 227 | 11.6 | 0.096 |
| 32.037 | 2.7915 | 115 | 5.9 | 0.192 |
| 33.545 | 2.6694 | 80 | 4.1 | 0.144 |
| 34.433 | 2.6025 | 8 | 0.4 | 0.288 |
| 35.658 | 2.5159 | 73 | 3.8 | 0.072 |
| 35.869 | 2.5015 | 34 | 1.8 | 0.072 |
| 36.744 | 2.4440 | 219 | 11.2 | 0.096 |

In a fourth aspect, the present application provides a method for preparing the crystal form B of proximod described in the third aspect. The method includes the following steps: adding the crystal form A of proximod described above to a mixed solvent, adjusting a pH of the system to 1-2 by adding an acid, heating to reflex until fully dissolved, cooling to 40 °C-50 °C, stirring, further cooling to room temperature, separating a solid and drying under reduced pressure to obtain the crystal form B.

Preferably, the pH of the system is adjusted by using an acid, and the acid is concentrated hydrochloric acid.

Preferably, the mixed solvent includes ethanol and water.

Preferably, a volume ratio of ethanol to water is 9: 1.

In the present application, natural cooling may be adopted for the cooling.

Preferably, the solid is dried under reduced pressure at a temperature of 40 °C-55 °C, for example, 45 °C, 48 °C, 50 °C, 53 °C or 55 °C.

Preferably, the seed of the crystal form B is added to the system after the natural cooling to 40 °C-50 °C°C°C. On the basis that the crystal form B is already obtained in the present application, when the crystal form B is prepared again, the seed of the crystal form B may be added during the preparation to facilitate the generation of the crystal form B.

In the present application, the crystal form A and the crystal form B can stably exist, have low hygroscopicity, high solubility, good mechanical stability, low fluidity, low adhesiveness, and good compressibility, and have pharmaceutical industrialization prospects. The crystal form A and the crystal form B are pH-dependent. By adjusting the preparation processes, the crystal form A and the crystal form B of proximod may be obtained under different pH conditions. With the crude sample of proximod as the starting material, the crystal form A of proximod may be obtained when the pH of the system is 6-6.5, and the crystal form B of proximod may be prepared from the crystal form A when the pH of the system is 1-2.

During the preparation of the crystal form B from the crystal form A, a mixed crystal is formed if the pH of the system exceeds 2, and degradation of the compound occurs if the pH is less than 1.

In the present application, the crystal form B is obtained by recrystallizing the crystal form A under the condition that the pH of the system is 1-2. If the crude sample of proximod is directly added to the mixed solvent, then the pH of the system is adjusted to 1-2, the temperature is raised to reflex to dissolve the crude sample to a clear solution, the temperature is lowered to 40 °C-50 °C, the solution is stirred, and the temperature is lowered to room temperature, the crystal form B cannot be formed.

In the present application, the crystal form A of proximod has lower hygroscopicity, higher solubility, better mechanical stability and fluidity than the crystal form B.

In the studies of the present application, it has been found that proximod has multiple crystal forms, including but not limited to a crystal form A, a crystal form B, a crystal form C, a crystal form D, a crystal form E, a crystal form F, and the like. Compared with other crystal forms, the crystal form A and the crystal form B are more stable, have lower hygroscopicity, higher solubility, better mechanical stability, lower fluidity, lower adhesiveness, and better compressibility, and have pharmaceutical industrialization prospects.

In a fifth aspect, the present application provides a pharmaceutical composition. The pharmaceutical composition includes the crystal form A of proximod described in the first aspect and/or the crystal form B of proximod described in the third aspect and a pharmaceutically acceptable adjuvant.

Preferably, the pharmaceutically acceptable adjuvant includes microcrystalline cellulose.

The microcrystalline cellulose used as the adjuvant in the present application can improve the compressibility of the crystal form A and the crystal form B of proximod so that the hardness and tensile strength of the resulting tablets can be improved.

Preferably, the pharmaceutical composition includes the crystal form A of proximod described above and a pharmaceutically acceptable adjuvant, and the pharmaceutically acceptable adjuvant includes microcrystalline cellulose.

The usage of microcrystalline cellulose can more significantly improve the compressibility of the crystal form A than the compressibility of the crystal form B so that the tablet formation performance of the crystal form A is better and the quality of the resulting tablets is higher.

In a sixth aspect, the present application provides use of the crystal form A of proximod described in the first aspect, the crystal form B of proximod described in the third aspect or the pharmaceutical composition described in the fifth aspect in the preparation of a drug having an immunomodulatory effect.

Preferably, the drug having the immunomodulatory effect is selected from drugs for treating immune disorders, hypoimmunity, immunosuppression, rejection reactions after organ transplantation or autoimmune diseases.

Preferably, the drug having the immunomodulatory effect is selected from drugs for treating rheumatoid arthritis.

Compared with the existing art, the present application has the following beneficial effects.

In the present application, the crystal form A and the crystal form B can stably exist, have low hygroscopicity, high solubility, good mechanical stability, low fluidity, low adhesiveness, and good compressibility, and have pharmaceutical industrialization prospects.

The crystal form A and the crystal form B in the present application have pH dependence. By adjusting the

preparation processes, the crystal form A and the crystal form B of proximod may be obtained under different pH conditions. With the crude sample of proximod as the starting material, the crystal form A of proximod may be obtained when the pH of the system is 6-6.5, and the crystal form B of proximod may be prepared when the pH of the system is 1-2.

The crystal form A of proximod has lower hygroscopicity, higher solubility, better mechanical stability, lower fluidity, and better compressibility than the crystal form B.

BRIEF DESCRIPTION OF DRAWINGS

FIG. 1 is an X-ray powder diffraction (XRPD) pattern of a crystal form A of proximod.

FIG. 2 is a differential scanning calorimetry (DSC) curve of the crystal form A of proximod.

FIG. 3 is a thermogravimetry (TGA) curve of the crystal form A of proximod.

FIG. 4 is a Fourier-transform infrared (FT-IR) spectroscopy of the crystal form A of proximod.

FIG. 5 is a dynamic vapor sorption (DVS) plot of the crystal form A of proximod.

FIG. 6 is an X-ray powder diffraction (XRPD) pattern of a crystal form B of proximod.

FIG. 7 is a differential scanning calorimetry (DSC) curve of the crystal form B of proximod.

FIG. 8 is a thermogravimetry (TGA) curve of the crystal form B of proximod.

FIG. 9 is a Fourier-transform infrared (FT-IR) spectroscopy of the crystal form B of proximod.

FIG. 10 is a dynamic vapor sorption (DVS) plot of the crystal form B of proximod.

FIG. 11 is an X-ray powder diffraction (XRPD) pattern of a crystal form C of proximod.

FIG. 12 is a differential scanning calorimetry (DSC) curve of the crystal form C of proximod.

FIG. 13 is a thermogravimetry (TGA) curve of the crystal form C of proximod.

FIG. 14 is an X-ray powder diffraction (XRPD) pattern of a crystal form D of proximod.

FIG. 15 is an X-ray powder diffraction (XRPD) pattern of the dried crystal form D of proximod.

FIG. 16 is a thermogravimetry (TGA) curve of the dried crystal form D of proximod.

FIG. 17 is a thermogravimetry (TGA) curve of the crystal form D of proximod which is dried and placed at room temperature for two days.

FIG. 18 is an X-ray powder diffraction (XRPD) pattern of a crystal form E of proximod.

FIG. 19 is a differential scanning calorimetry (DSC) curve of the crystal form E of proximod.

FIG. 20 is a thermogravimetry (TGA) curve of the crystal form E of proximod.

FIG. 21 is an X-ray powder diffraction (XRPD) pattern of a crystal form F of proximod.

FIG. 22 is an X-ray powder diffraction (XRPD) pattern of the dried crystal form F of proximod.

FIG. 23 is an X-ray powder diffraction (XRPD) overlay pattern of the crystal form A of proximod which is stably placed.

FIG. 24 is an X-ray powder diffraction (XRPD) overlay pattern of the crystal form B of proximod which is stably placed.

FIG. 25 is an X-ray powder diffraction (XRPD) overlay pattern of the crystal form A of proximod before and after milling.

FIG. 26 is an X-ray powder diffraction (XRPD) overlay pattern of the crystal form B of proximod before and after milling.

FIG. 27 is an X-ray powder diffraction (XRPD) overlay pattern of the crystal form A of proximod before and after compression.

FIG. 28 is an X-ray powder diffraction (XRPD) overlay pattern of the crystal form B of proximod before and after compression.

FIG. 29 is a microscopy observation result of the crystal form A of proximod, where the scale is 150 μm.

FIG. 30 is a microscopy observation result of the crystal form B of proximod, where the scale is 50 μm.

DETAILED DESCRIPTION

Technical solutions of the present application are further described below through specific examples. Those skilled in the art are to understand that the examples described herein are used for a better understanding of the present application and are not to be construed as specific limitations to the present application.

In the examples of the present application, the instrument used for the X-ray powder diffraction test is the D2 Phaser produced by Bruker. The instrument used for the differential scanning calorimetry test is the differential scanning calorimeter DSC 250 produced by TA Instruments. The instrument used for the thermogravimetric analysis is the thermogravimetric analyzer TGA 55 produced by TA Instruments. The instrument used for the FT-IR test is the Fourier infrared spectrometer ID1-summit produced by Thermo. The instrument used for the dynamic vapor sorption test is the DVS Intrinsic produced by Surface Measurement Systems (SMS). The instrument used for the microscopy is the microscope NJF-120A produced by Suzhou Nanguang Electronic Technology Co., Ltd.

**Example 1 Preparation of Crystal Form A of Proximod**

0.5 g of crude sample of proximod was loaded into a 3 mL glass bottle, 2 mL of tetrahydrofuran/water (9:1, v/v) was added, the pH of the system was kept at 6-6.5, and the temperature was raised to dissolve the crude sample to prepare a clear solution for use. The clear solution was added dropwise to 10 mL of methyl *tert*-butyl ether and magnetically stirred at room temperature for 30 minutes, the resulting solid was separated and dried at 45 °C under reduced pressure overnight to obtain 0.42 g of solid (with a yield of 84.0%). The obtained solid was tested by the XRPD detection and confirmed as the crystal form A.

FIG. 1 shows the XRPD pattern of the separated crystal form A of 2-amino-2-{2-(4'-(2-propyloxazol-4-yl)-[1,1'-biphenyl]-4-yl)ethyl}-1,3-propanediol hydrochloride, and the XRPD parameters in the figure are summarized in Table 1 below.

Table 1 XRPD parameters of the crystal form A

| 2θ (°) | d (Å) | Height (counts) | $I/I_0$ (%) | FWHM (°) |
|---|---|---|---|---|
| 3.208 | 27.5164 | 9163 | 39.5 | 0.096 |
| 5.166 | 17.0908 | 22 | 0.1 | 0.072 |
| 6.080 | 14.5260 | 28 | 0.1 | 0.072 |
| 6.426 | 13.7427 | 598 | 2.6 | 0.120 |
| 6.835 | 12.9221 | 30 | 0.1 | 0.072 |
| 9.688 | 9.1222 | 23173 | 100.0 | 0.096 |
| 12.033 | 7.3489 | 7 | 0.0 | 0.192 |
| 12.946 | 6.8329 | 2002 | 8.6 | 0.120 |
| 16.195 | 5.4686 | 739 | 3.2 | 0.072 |
| 17.037 | 5.2002 | 55 | 0.2 | 0.144 |
| 18.512 | 4.7891 | 38 | 0.2 | 0.072 |
| 19.483 | 4.5526 | 11154 | 48.1 | 0.120 |

(continued)

| 2θ (°) | d (Å) | Height (counts) | $I/I_0$ (%) | FWHM (°) |
|---|---|---|---|---|
| 20.927 | 4.2416 | 127 | 0.6 | 0.096 |
| 23.778 | 3.7390 | 3306 | 14.3 | 0.120 |
| 25.375 | 3.5072 | 129 | 0.6 | 0.216 |
| 26.116 | 3.4094 | 439 | 1.9 | 0.120 |
| 26.500 | 3.3608 | 1305 | 5.6 | 0.072 |
| 26.845 | 3.3184 | 1170 | 5.1 | 0.096 |
| 27.619 | 3.2272 | 16 | 0.1 | 0.072 |
| 29.402 | 3.0354 | 402 | 1.7 | 0.120 |
| 30.085 | 2.9680 | 681 | 2.9 | 0.096 |
| 32.774 | 2.7303 | 472 | 2.0 | 0.096 |
| 33.335 | 2.6857 | 245 | 1.1 | 0.120 |
| 33.569 | 2.6675 | 25 | 0.1 | 0.072 |
| 33.832 | 2.6474 | 9 | 0.0 | 0.144 |
| 34.226 | 2.6178 | 127 | 0.6 | 0.120 |
| 36.731 | 2.4448 | 240 | 1.0 | 0.120 |
| 37.132 | 2.4193 | 94 | 0.4 | 0.072 |
| 38.279 | 2.3494 | 9 | 0.0 | 0.144 |

The crystal form A has a differential scanning calorimetry (DSC) curve shown in FIG. 2. As can be seen from FIG. 2, the first endothermic peak occurs when the crystal form A is heated to 188 °C (onset temperature), and the endothermic peak is a melting endothermic peak; the second endothermic peak occurs when the crystal form A is heated to 214 °C (onset temperature), and the endothermic peak is a decomposition endothermic peak.

The crystal form A has a thermogravimetry (TGA) curve shown in FIG. 3. As can be seen from FIG. 3, the crystal form A has a weight loss of about -0.2% when the crystal form A was heated to 150 °C (with the instrument error of ± 0.2%).

The crystal form A has a Fourier-transform infrared (FT-IR) spectroscopy shown in FIG. 4. As can be seen from FIG. 4, the infrared spectrum qualitative data may be obtained by characterizing the crystal form A by attenuated total reflectance-Fourier transform infrared. The important bands described below (expressed as the reciprocal of wavelength data ($cm^{-1}$)) are used for qualification, and the data are shown in Table 2 below.

Table 2

| Wavelength reciprocal ($cm^{-1}$) | Transmittance (%) | Wavelength reciprocal ($cm^{-1}$) | Transmittance (%) |
|---|---|---|---|
| 519 | 82 | 1174 | 86 |
| 610 | 92 | 1249 | 89 |
| 764 | 73 | 1297 | 86 |
| 817 | 62 | 1316 | 88 |
| 841 | 84 | 1402 | 81 |
| 941 | 76 | 1464 | 76 |
| 958 | 87 | 1491 | 50 |
| 1005 | 85 | 1515 | 71 |
| 1027 | 71 | 1581 | 65 |
| 1052 | 68 | 1608 | 81 |
| 1069 | 61 | 2963 | 47 |
| 1095 | 82 | 3347 | 57 |

(continued)

| Wavelength reciprocal ($cm^{-1}$) | Transmittance (%) | Wavelength reciprocal ($cm^{-1}$) | Transmittance (%) |
|---|---|---|---|
| 1118 | 78 | 3565 | 63 |

The crystal form A has a dynamic vapor sorption (DVS) plot shown in FIG. 5. As can be seen from FIG. 5, the sample absorbs water, gains weight by 0.26%, and thus is slightly hygroscopic (vapor sorption at 25 °C/ 90% RH).

**Comparative Example 1**

Comparative Example 1 differs from Example 1 only in that the pH of the system was kept at 5.0 during the preparation process, and the rest of the steps are the same as those of Example 1. After analysis, the mixed crystal of A and B was formed.

**Comparative Example 2**

Comparative Example 2 differs from Example 1 only in that the pH of the system was kept at 7.0 during the preparation process, and the rest of the steps are the same as those of Example 1. No crystal was formed.

**Comparative Example 3**

Comparative Example 3 differs from Example 1 only in that the step of dropwise adding the dissolved clear solution to methyl tert-butyl ether was not included in the preparation process. After analysis, the mixed crystal of A and B was formed.

**Example 2 Preparation of Crystal Form B of Proximod**

4.0 g of sample of the crystal form A of proximod was loaded into an 80 mL glass bottle, 48.0 mL of a mixed solvent of ethanol/water (9:1, v/v) was added, concentrated hydrochloric acid was added to adjust the pH of the system to 1-2, and the temperature was raised to reflux to dissolve the sample to prepare a clear solution. The system was naturally cooled to 45 °C under magnetic stirring, stirred for 30 minutes, naturally cooled to room temperature, and then stirred continuously. The solid was separated and dried under vacuum at 50 °C overnight to obtain 2.99 g of the crystal form B as an off-white solid (with a yield of 74.8%). The obtained solid was tested by the XRPD detection and confirmed as the crystal form B of the present application.

FIG. 6 shows the XRPD pattern of the separated crystal form B of 2-amino-2-{2-(4'-(2-propyloxazol-4-yl)-[1,1'-biphenyl]-4-yl)ethyl}-1,3-propanediol hydrochloride, and the XRPD parameters are summarized in Table 3 below.

Table 3 XRPD parameters of the crystal form B

| 2θ (°) | d (Å) | Height (counts) | $I/I_0$ (%) | FWHM (°) |
|---|---|---|---|---|
| 4.495 | 19.6424 | 1074 | 54.9 | 0.096 |
| 7.794 | 11.3345 | 47 | 2.4 | 0.072 |
| 8.670 | 10.1906 | 40 | 2.0 | 0.072 |
| 9.009 | 9.8078 | 1001 | 51.2 | 0.120 |
| 9.350 | 9.4510 | 32 | 1.6 | 0.072 |
| 9.708 | 9.1035 | 308 | 15.8 | 0.096 |
| 10.215 | 8.6531 | 20 | 1.0 | 0.072 |
| 10.775 | 8.2040 | 153 | 7.8 | 0.120 |
| 11.206 | 7.8894 | 20 | 1.0 | 0.144 |
| 12.018 | 7.3584 | 242 | 12.4 | 0.144 |
| 13.575 | 6.5178 | 912 | 46.7 | 0.096 |
| 14.770 | 5.9930 | 10 | 0.5 | 0.120 |
| 15.388 | 5.7536 | 75 | 3.8 | 0.072 |

(continued)

| 2θ (°) | d (Å) | Height (counts) | $I/I_0$ (%) | FWHM (°) |
|---|---|---|---|---|
| 15.720 | 5.6329 | 19 | 1.0 | 0.072 |
| 16.138 | 5.4878 | 230 | 11.8 | 0.096 |
| 16.542 | 5.3547 | 16 | 0.8 | 0.072 |
| 17.528 | 5.0556 | 274 | 14.0 | 0.120 |
| 17.821 | 4.9732 | 167 | 8.6 | 0.120 |
| 18.138 | 4.8870 | 448 | 22.9 | 0.096 |
| 18.532 | 4.7838 | 320 | 16.4 | 0.120 |
| 19.258 | 4.6052 | 1279 | 65.4 | 0.096 |
| 19.538 | 4.5398 | 354 | 18.1 | 0.096 |
| 20.553 | 4.3178 | 216 | 11.0 | 0.096 |
| 20.994 | 4.2281 | 92 | 4.7 | 0.096 |
| 21.541 | 4.1221 | 390 | 20.0 | 0.072 |
| 21.764 | 4.0803 | 1956 | 100.0 | 0.120 |
| 22.336 | 3.9771 | 290 | 14.8 | 0.096 |
| 22.733 | 3.9085 | 209 | 10.7 | 0.096 |
| 24.239 | 3.6690 | 294 | 15.0 | 0.144 |
| 24.531 | 3.6259 | 875 | 44.8 | 0.096 |
| 25.527 | 3.4867 | 205 | 10.5 | 0.120 |
| 25.733 | 3.4592 | 172 | 8.8 | 0.096 |
| 26.464 | 3.3653 | 83 | 4.2 | 0.120 |
| 27.469 | 3.2444 | 468 | 23.9 | 0.096 |
| 27.755 | 3.2117 | 670 | 34.3 | 0.072 |
| 30.364 | 2.9414 | 437 | 22.4 | 0.120 |
| 31.098 | 2.8735 | 231 | 11.8 | 0.072 |
| 31.413 | 2.8455 | 227 | 11.6 | 0.096 |
| 32.037 | 2.7915 | 115 | 5.9 | 0.192 |
| 33.545 | 2.6694 | 80 | 4.1 | 0.144 |
| 34.433 | 2.6025 | 8 | 0.4 | 0.288 |
| 35.658 | 2.5159 | 73 | 3.8 | 0.072 |
| 35.869 | 2.5015 | 34 | 1.8 | 0.072 |
| 36.744 | 2.4440 | 219 | 11.2 | 0.096 |

The crystal form B has a differential scanning calorimetry (DSC) curve shown in FIG. 7. As can be seen from FIG. 7, the first endothermic peak occurs when the crystal form B is heated to 173.9 °C (onset temperature), and the endothermic peak is a melting endothermic peak; the second endothermic peak occurs when the crystal form B is heated to 213.1 °C (onset temperature), and the endothermic peak is a decomposition endothermic peak.

The crystal form B has a thermogravimetry (TGA) curve shown in FIG. 8. As can be seen from FIG. 8, the crystal form B has a weight loss of about -0.2% when the crystal form B was heated to 150 °C (with the instrument error of ± 0.2%). The TGA results indicate that the crystal form B is anhydrous.

The crystal form B has a Fourier-transform infrared (FT-IR) spectroscopy shown in FIG. 9. As can be seen from FIG. 9, the infrared spectrum qualitative data may be obtained by characterizing the crystal form B by attenuated total reflectance-Fourier transform infrared. The important bands described below (expressed as the reciprocal of wavelength

data ($cm^{-1}$)) are used for qualification, and the data are shown in Table 4 below.

Table 4

| Wavelength reciprocal ($cm^{-1}$) | Transmittance (%) | Wavelength reciprocal ($cm^{-1}$) | Transmittance (%) |
|---|---|---|---|
| 518 | 93 | 1453 | 90 |
| 765 | 84 | 1494 | 86 |
| 812 | 83 | 1533 | 87 |
| 840 | 95 | 1571 | 87 |
| 946 | 87 | 1595 | 86 |
| 1020 | 80 | 2532 | 89 |
| 1064 | 83 | 2844 | 77 |
| 1117 | 90 | 2930 | 78 |
| 1198 | 95 | 2967 | 78 |
| 1286 | 91 | 3026 | 81 |
| 1339 | 93 | 3181 | 78 |
| 1402 | 91 | | |

The crystal form B has a dynamic vapor sorption (DVS) plot shown in FIG. 10. As can be seen from FIG. 10, the sample absorbs water, gains weight by 0.26%, and thus is slightly hygroscopic (vapor sorption at 25 °C/90% RH).

**Comparative Example 4**

Comparative Example 4 differs from Example 2 only in that concentrated hydrochloric acid was added to adjust the pH of the system to 3, and the rest of the steps are the same as those of Example 2. After analysis, the mixed crystal of A and B was formed.

**Comparative Example 5**

Comparative Example 5 differs from Example 2 only in that concentrated hydrochloric acid was added to adjust the pH of the system to 0.5, and the rest of the steps are the same as those of Example 2. After analysis, degradation of the compound occurred under these conditions.

**Comparative Example 6**

4.0 g of crude sample of proximod was loaded into an 80 mL glass bottle, 48.0 mL of a mixed solvent of ethanol/water (9:1, v/v) was added, concentrated hydrochloric acid was added to adjust the pH of the system to 1-2, and the temperature was raised to reflux to dissolve the crude sample to prepare a clear solution. The system was naturally cooled to 45 °C under magnetic stirring, stirred for 30 minutes, naturally cooled to room temperature, and then stirred continuously. The solid was separated and dried under vacuum at 50 °C overnight. After analysis, the mixed crystal of A and B was formed.

**Example 3 Preparation of Crystal Form C of Proximod**

38 mg of the sample of the crystal form A of proximod was placed in an aluminum pan, and in the thermogravimetric analyzer TGA 55 produced by TA Instruments, the sample was heated to 200 °C at a heating rate of 10 °C/minute, kept at a constant temperature with a nitrogen purge for 40 minutes, and then cooled to room temperature to obtain a light yellow solid.

The sample heated to 200 °C was detected by ion chromatography, and the chloride ion content was 8.05% (the theoretical chloride ion content is 8.51%). The heated sample was tested for purity, and the HPLC purity test result was 98.42%. The above results indicate that the heating operation does not lead to significant decomposition of proximod.

After the XRPD detection, the X-ray powder diffraction (XRPD) pattern of the crystal form C is shown in FIG. 11, and the obtained solid was the crystal form C of the present application. The crystal form C of proximod is prepared in the

heating experiment and is difficult to industrialize. The DSC curve of the crystal form C is shown in FIG. 12. As can be seen from FIG. 12, the first endothermic peak occurs when the crystal form C is heated to 114.1 °C (onset temperature), and the endothermic peak is a melting endothermic peak; one exothermic peak occurs when the crystal form C is heated to 137.5 °C (onset temperature), and the exothermic peak is a crystal transformation exothermic peak; the second endothermic peak occurs when the crystal form C is heated to 184.2 °C (onset temperature), and the endothermic peak is a melting endothermic peak; the third endothermic peak occurs when the crystal form C is heated to 211.8 °C (onset temperature), and the endothermic peak is a decomposition endothermic peak. The TGA curve is shown in FIG. 13, and the weight loss of the sample was 1.4% before 150 °C. The heated sample was detected by ion chromatography, and the chloride ion content was 8.05% (the theoretical chloride ion content is 8.51%). The HPLC purity test result was 98.42%. The above results indicate that the heating operation does not lead to significant decomposition of proximod. Based on the above characterization results, it is inferred that the crystal form C is anhydrous.

**Example 4 Preparation of Crystal Form D of Proximod**

500 mg of the sample of the crystal form A of proximod was loaded into a 10 mL glass bottle, 5 mL of 1,4-dioxane was added to form a suspension, the suspension was stirred at room temperature for 72 hours, and a white solid was separated. After the XRPD detection, FIG. 14 was obtained, and it indicates that the obtained solid is the crystal form D of the present application.

After the above sample was dried under vacuum at 50 °C for 6 hours, the XRPD detection was performed. As shown in FIG. 15, the solid was partially crystal transformed into the crystal form A of the present application. The TGA curves of the sample of the crystal form D of proximod which was dried and the sample of the crystal form D of proximod which was dried and placed at room temperature for two days are shown in FIGS. 16 and 17, respectively. As can be seen, the sample of the dried crystal form D was subjected to the TGA test and had a weight loss of about 5.4% (as shown in FIG. 16) when heated to 150 °C; the sample of the crystal form D which was dried and placed at room temperature for two days was subjected to the TGA test and had a weight loss of about 4.8% (as shown in FIG. 17) when heated to 150 °C. The theoretical weight loss of the proximod hemihydrate is 6.1%. As can be seen from the TGA results, the crystal water of the crystal form D is continuously lost during storage. Based on the above experimental results, it is inferred that the crystal form D is an unstable sesquihydrate. It is inferred that the crystal form D is an unstable sesquihydrate and is prone to crystal transformation during drying and storage, making it difficult to prepare a pure phase.

**Example 5 Preparation of Crystal Form E of Proximod**

50 mg of the sample of the crystal form A of proximod was loaded into a 20 mL glass bottle, 10 mL of water was added to form a suspension, the suspension was magnetically stirred at room temperature for 24 hours, and a white solid was separated. After the XRPD detection, the obtained solid was the crystal form E of the present application, and the X-ray powder diffraction (XRPD) pattern of the crystal form E is shown in FIG. 18. The crystal form E of proximod is prepared by chance in the heating experiment and is difficult to repeatedly prepare. The thermogravimetric graph of the crystal form E of proximod is shown in FIG. 19, and the TGA curve of the crystal form E of proximod is shown in FIG. 20. The TGA results indicate that the crystal form E is anhydrous.

**Example 6 Preparation of Crystal Form F of Proximod**

50 mg of the sample of the crystal form A of proximod was loaded into a 20 mL glass bottle, 10 mL of water was added to form a suspension, the suspension was magnetically stirred at 50 °C for 24 hours, and a white solid was separated. The obtained wet sample was subjected to the XRPD detection. The obtained solid was the crystal form F of the present application, and the X-ray powder diffraction (XRPD) pattern of the crystal form F is shown in FIG. 21. After the wet sample was dried under vacuum at 50 °C for 2 hours, the XRPD detection was performed. The results are shown in FIG. 22, and the dried sample was partially crystal transformed into the crystal form A. Therefore, as can be seen, the crystal form F is a metastable crystal form, making it difficult to prepare a pure phase.

**Example 7 Solid-State Stability of Crystal Form A and Crystal Form B of Proximod**

Through the polymorphic screening of proximod, a total of 6 crystal forms are found, named the crystal form A, the crystal form B, the crystal form C, the crystal form D, the crystal form E, and the crystal form F. The crystal form C is an anhydrous crystal form obtained by heating experiments and is difficult to industrialize. The crystal form D is a hydrate crystal form, but it is unstable and is prone to water loss during storage, making it difficult to obtain a pure phase. The crystal form E is an anhydrous crystal form obtained by chance, making it difficult to repeatedly prepare. The crystal form F is a metastable crystal form of unknown type and is prone to crystal transformation into the crystal form A, making it difficult to

obtain a pure phase. To sum up, the crystal form A and crystal form B of proximod have industrial application values.

Samples of the crystal form A and the crystal form B were stored at 60 °C, at 92.5% RH, in light, and at 45 °C/75% RH, respectively. The crystal forms of the samples before and after storage were tested by XRPD. The results are shown in Table 5, and the XRPD comparison charts are shown in FIG. 23 and FIG. 24. The results indicate that the crystal forms of the crystal form A and the crystal form B could remain stable for at least 30 days at 60 °C, 92.5% RH, and 45 °C/75% RH; the main purity showed no notable variation after storage at 40 °C/75% RH, 60 °C, and 92.5% RH for 30 days and decreased after storage in light for 30 days.

Table 5 Stability study on the crystal form A and the crystal form B

| Storage condition | Crystal form/purity (A%) | |
|---|---|---|
| Storage condition | Crystal form/purity (A%) | |
| | Before storage | After storage |
| At 40 °C/75% RH, 30 days, exposed | Crystal form A<br>Main purity: 99.79<br>Maximum single impurity: 0.04 | Crystal form A<br>Main purity: 99.82<br>Maximum single impurity: 0.04 |
| At 60 °C, 30 days, exposed | | Crystal form A<br>Main purity: 99.80<br>Maximum single impurity: 0.04 |
| In light, 30 days, exposed | | Crystal form A<br>Main purity: 98.83<br>Maximum single impurity: 0.43 |
| At 92.5%RH, 30 days, exposed | | Crystal form A<br>Main purity: 99.81<br>Maximum single impurity: 0.04 |
| At 40 °C/75%RH, 30 days, exposed | Crystal form B<br>Main purity: 99.78<br>Maximum single impurity: 0.04 | Crystal form B<br>Main purity: 99.80<br>Maximum single impurity: 0.04 |
| At 60 °C, 30 days, exposed | | Crystal form B<br>Main purity: 99.79<br>Maximum single impurity: 0.04 |
| In light, 30 days, exposed | | Crystal form B<br>Main purity: 99.19<br>Maximum single impurity: 0.21 |
| At 92.5%RH, 30 days, exposed | | Crystal form B<br>Main purity: 99.83<br>Maximum single impurity: 0.04 |

**Example 8 Dynamic Solubility Study on Crystal Form A and Crystal Form B of Proximod**

Samples of the crystal form A and the crystal form B of the present application were prepared into saturated solutions at 37 °C with a fasted state simulated intestinal fluid (FaSSIF, at a pH of 8.0), a fed state simulated intestinal fluid (FeSSIF, at a pH of 5.0), a simulated gastric fluid (SGF, at a pH of 1.0), a pH = 1.0 hydrochloric acid aqueous solution, a pH = 4.0 acetic acid-sodium acetate buffer system, a pH = 6.8 phosphate buffer system, and water, respectively. After 1 hour/2 hours/4 hours/24 hours, the saturated solutions were obtained by filtration. The actual concentrations of the samples in the saturated solutions were determined by high-performance liquid chromatography (HPLC) (the filtered solutions were diluted as needed), and the crystal forms of the solids before and after the experiment were characterized by XRPD to check whether phase changes occurred. The experimental results are shown in FIG. 6.

Table 6 Stability of the crystal form A and the crystal form B

| Medium | Sampling point | | 1 hour | 2 hours | 4 hours | 24 hours |
|---|---|---|---|---|---|---|
| pH = 1.0 | Crystal form A | Solubility (mg/mL) | 0.28 | 0.28 | 0.33 | 0.40 |
| | | Crystal form | A + F | A + F | A + F | A + F |
| | Crystal form B | Solubility (mg/mL) | 0.27 | 0.28 | 0.28 | 0.30 |
| | | Crystal form | B | B | B | B |
| pH = 4.0 | Crystal form A | Solubility (mg/mL) | 1.69 | 1.13 | 1.36 | 4.11 |
| | | Crystal form | A + F | A + F | A + F | A + F |
| | Crystal form B | Solubility (mg/mL) | 1.91 | 2.35 | 1.99 | 2.31 |
| | | Crystal form | B + F | B + F | B + F | B + F |
| pH = 6.8 | Crystal form A | Solubility (mg/mL) | 0.00016 | Not detected | Not detected | Not detected |
| | | Crystal form | Free alkali | Free alkali | Free alkali | Free alkali |
| | Crystal form B | Solubility (mg/mL) | Not detected | 0.00028 | Not detected | Not detected |
| | | Crystal form | Free alkali | Free alkali | Free alkali | Free alkali |
| SGF | Crystal form A | Solubility (mg/mL) | 0.26 | 0.25 | 0.28 | 0.46 |
| | | Crystal form | A + F | A + F | A + F | A + F |
| | Crystal form B | Solubility (mg/mL) | 0.31 | 0.27 | 0.29 | 0.31 |
| | | Crystal form | B | B | B | B |
| FaSSIF | Crystal form A | Solubility (mg/mL) | 0.022 | 0.024 | 0.018 | 0.016 |
| | | Crystal form | A + Free alkali | A + Free alkali | Free alkali | Free alkali |
| | Crystal form B | Solubility (mg/mL) | 0.070 | 0.20 | 0.41 | 0.10 |
| | | Crystal form | Free alkali | Free alkali | Free alkali | Free alkali |
| FeSSIF | Crystal form A | Solubility (mg/mL) | 1.93 | 2.11 | 0.34 | 3.72 |
| | | Crystal form | A | A | A | A |
| | Crystal form B | Solubility (mg/mL) | 2.58 | 2.30 | 2.56 | 3.46 |
| | | Crystal form | B | B | B | B |
| Water | Crystal form A | Solubility (mg/mL) | 2.02 | 2.57 | 0.99 | 3.29 |
| | | Crystal form | A + F | A + F | A + F | A + F |
| | Crystal form B | Solubility (mg/mL) | 1.91 | 2.43 | 2.24 | 2.61 |
| | | Crystal form | B + F | B + F | B + F | B + F |

The results indicate that the solubility of the crystal form A in the FeSSIF, the SGF, the pH = 1.0 hydrochloric acid aqueous solution, the pH = 4.0 acetic acid-sodium acetate buffer system, and water is higher than the solubility of the crystal form B. After stirred in the FeSSIF at 37 °C for 24 hours, the crystal form A and the crystal form B showed no crystal form change. The above results indicate that the crystal form A is an advantageous crystal form suitable for drug development.

**Example 9 Equilibrium Solubility Study on Crystal Form A and Crystal Form B of Proximod**

The crystal form A and the crystal form B of the present application were prepared into saturated solutions with ethanol, acetone, and ethyl acetate at room temperature, respectively. After 24 hours of equilibrium, the saturated solutions were obtained by filtration. The contents of the samples in the saturated solutions were determined by high-performance liquid chromatography (HPLC) (the filtered solutions were diluted as needed), and the crystal forms of the solids before and after the experiment were characterized by XRPD to check whether phase changes occurred. The experimental results are shown in FIG. 7. The results indicate that the solubility of the crystal form A in ethanol, acetone, and ethyl acetate is higher than the solubility of the crystal form B.

Table 7 Equilibrium solubility of the crystal form A and the crystal form B

| Starting crystal form \ Medium | | Ethanol | Acetone | Ethyl acetate |
|---|---|---|---|---|
| Crystal form A | Solubility (mg/mL) | 9.99 | 0.043 | 0.011 |
| | Solid-state crystal form | A | A | A |
| Crystal form B | Solubility (mg/mL) | 7.61 | 0.032 | Not detected |
| | Solid-state crystal form | B | B | B |

**Example 10 Intrinsic Dissolution of Crystal Form A and Crystal Form B of Proximod**

The crystal form A and the crystal form B of the present application were prepared by tableting to determine the dissolution. The intrinsic dissolution results of the crystal form A and the crystal form B of proximod are shown in Table 8. The results indicate that the dissolution amounts of the two crystal forms in the medium of water are extremely low; in the medium with pH = 4.0 (the medium is an acetic acid-sodium acetate buffer system and is prepared in the following method: 1.22 g of sodium acetate and 20.5 mL of 2 mol/L acetic acid solution are dissolved in water, diluted to 1000 mL, and shaken well), the two crystal forms can be rapidly released at a low flow rate of 4 mL/minute, and through the comparison between the dissolution rates in the first 30 minutes, the crystal form A is released faster, showing the advantages in pharmaceutical production.

Table 8 Intrinsic solubility of the crystal form A and the crystal form B

| Crystal form | Medium | Dissolution amount (mg/mL) | | | | | |
|---|---|---|---|---|---|---|---|
| | | 5 min | 10 min | 30 min | 45 min | 60 min | 90 min |
| Crystal form A | pH = 4.0 | 0.0346 | 0.0462 | 0.0481 | 0.0477 | 0.0487 | 0.0479 |
| Crystal form B | | 0.0307 | 0.0404 | 0.0461 | 0.0488 | 0.0469 | 0.0487 |
| Crystal form A | Water | 0.0005 | 0.0010 | 0.0011 | 0.0003 | 0.0010 | 0.0012 |
| Crystal form B | | Not detected | 0.0002 | 0.0003 | 0.0009 | 0.0003 | 0.0004 |

**Example 11 Morphological Study on Crystal Form A and Crystal Form B of Proximod**

The microscopic morphology of the samples of the crystal form A and the crystal form B of the present application was studied, and the microscopy observation results are shown in FIG. 29 and FIG. 30. The crystal form A is flaky, the particle size is relatively larger and mostly ranges from 100 μm to 300 μm, and the length/short diameter ratio is 1-3; The crystal form B is rod-shaped, the maximum particle size is less than 100 μm, the particle size mainly ranges from 20 μm to 50 μm, and the length/short diameter ratio is 1-6.

**Example 12 Mechanical Stability of Crystal Form A and Crystal Form B of Proximod**

50 mg of the crystal form A and 50 mg of the crystal form B of the present application were ground clockwise with an agate mortar for 5 minutes, receptively. The samples before and after ground were detected by XRPD. The results are shown in FIG. 25 and FIG. 26. The XRPD results indicate that the crystal form of the crystal A remains unchanged after ground and the crystal form B is partially crystal transformed into the crystal form A after ground. The mechanical stability of the crystal form A is better than the mechanical stability of the crystal form B.

**Example 13 Hygroscopicity of Crystal Form A and Crystal Form B of Proximod**

Samples of the crystal form A and the crystal form B of the present application were subjected to dynamic moisture adsorption tests. The results are shown in FIG. 5 and FIG. 10. The test results indicate that the crystal form A has no or almost no hygroscopicity (which absorbs water and gains weight by 0.06% at 24.4 °C/90% RH), and the crystal form B is slightly hygroscopic (which absorbs water and gains weight by 0.30% at 25.6 °C/90% RH).

**Example 14 Fluidity of Crystal Form A and Crystal Form B of Proximod**

Since the powder properties of the drug substance affect the fluidity of the drug substance, the angles of repose and the Hausner ratios of the crystal form A and the crystal form B of proximod were measured to compare their fluidities. The fluidity evaluation results are shown in Table 9. The difference in adsorption rates between the two crystal forms is very small, and the fluidity of the crystal form A is slightly better than the fluidity of the crystal form B.

The bulk density and the tap density were measured in the following methods.

This experiment was performed by using a tap density tester according to the fixed-volume method. The specific operations are as follows: The weighed powder, with a volume of 50 mL, was loaded into a graduated cylinder, and the graduated cylinder was secured onto a support. The graduated cylinder was set to be vibrated at 55 ± 5 vibrations per minute for 6-8 minutes. The volume change of the powder was measured in a measuring cup. The ratio of the weight of the powder to the volume (50 mL) before tapping is the bulk density of the powder, and the ratio of the weight of the powder to the volume after tapping is the tapped density of the powder.
The Hausner ratio and the compression degree were converted in the following methods. The description of the Hausner ratio and the liquidity is shown in Table 10.

$$\text{Hausner ratio} = \text{tap density}/\text{bulk density}$$

$$\text{Compression degree} = (\text{tap density} - \text{bulk density})/\text{tap density}$$

The angle of repose was measured in the following method.
This experiment was performed by a pouring method, and the angles of repose of the drug substances of the two crystal forms were measured in combination with a residual cone method to analyze their fluidities. The specific operations are as follows: The powder was poured onto the center of a disk with a specific diameter, and when the material on the slope of the powder pile began to flow freely out along the periphery of the disk, the pouring of the powder was stopped. The height (h) of the cone was measured, and the angle of repose was calculated in combination with the diameter (2r) of the disk, that is, tanQ = h/r.

Table 9 Fluidity parameters of the crystal form A and the crystal form B

| Crystal form | Angle of repose (°) | Bulk density ($g/cm^3$) | Tap density ($g/cm^3$) | Hausner ratio | Compression degree |
|---|---|---|---|---|---|
| Crystal form A | 56.25 | 0.34 | 0.50 | 1.5 | 33.0% 33.0% |
| Crystal form B | 64.95 | 0.14 | 0.33 | 2.4 | 58.0% |

Table 10 Relationship between the Hausner ratio and the fluidity

| Hausner ratio | Fluidity description |
|---|---|
| 1.00-1.11 | The fluidity is very good |
| 1.12-1.18 | The fluidity is good |
| 1.19-1.25 | The fluidity is fair or moderate |
| 1.26-1.34 | The fluidity is not bad |
| 1.35-1.45 | The fluidity is bad |
| 1.46-1.59 | The fluidity is very bad |

(continued)

| Hausner ratio | Fluidity description |
|---|---|
| >1.60 | The fluidity is extremely bad |

**Example 15 Adhesiveness of Crystal Form A and Crystal Form B of Proximod**

The crystal form A and the crystal form B of the present application were loaded into fixed-size ziplock bags, respectively. The bags were sealed, flipped at a rotational speed of 5 rpm for 10 minutes, and continued to be flipped in a different flipping direction at a fixed rotational speed for 10 minutes. The materials were poured out of the bags, and the materials remaining in the bags were accurately weighed to calculate the adhesive rate.

The adhesive rate was calculated according to the following formula:

$$\text{Adhesive rate} = (\text{initial material weight} - \text{remaining material weight})/\text{contact surface area}.$$

The adhesiveness results of the crystal form A and the crystal form B of proximod are shown in Table 11. As can be seen from the results, the adhesive rates of the crystal form A and the crystal form B are equivalent.

Table 11 Adhesiveness of the crystal form A and the crystal form B

| Crystal form | PE bag adhesive rate ($g/cm^2$) |
|---|---|
| Crystal form A | 0.0007 |
| Crystal form B | 0.0003 |

**Example 16 Compressibility of Crystal Form A and Crystal Form B of Proximod**

The crystal form A and the crystal form B of the present application were tableted into preparation for evaluation. In this experiment, a die with a diameter of 6 mm was used. The drug substance and the drug substance + excipient were tableted under the same pressure, respectively. After the elasticity of the prepared tablets was restored, the radial crushing force F, diameter D, and thickness L of the tablets were measured. The tensile strength of the tablets was calculated according to the following formula: Ts = 2F/IIDL.

The compressibility evaluation results of the crystal form A and the crystal form B of proximod are shown in Table 12. The XRPD results before and after compression are shown in FIG. 27 and FIG. 28. As can be seen from the results, the materials of the two crystal forms have poor compressibility and exhibit severe punch sticking and difficulty in tablet formation; the compressibility of the material of the crystal form A can be significantly improved when the crystal form A is mixed with an excipient with good compressibility, so the compressibility of the crystal form A is better than the compressibility of the crystal form B.

Table 12 Compressibility of the crystal form A and the crystal form B

| No | Ingredient | Hardness/ N | Tensile strength Ts | Experimental phenomena |
|---|---|---|---|---|
| 1 | Crystal form A | 5 | 0.17 | Severe punch sticking |
| | Crystal form B | 3 | 0.10 | Severe punch sticking |
| 2 | Crystal form A + microcrystalline cellulose (100 mg + 100 mg) | 11 | 0.36 | Slight punch sticking |
| | Crystal form B + microcrystalline cellulose (100 mg + 100 mg) | 4 | 0.12 | Slight punch sticking |

The applicants have stated that although the crystal form of proximod, the preparation method therefor, and the use thereof in the present application have been described through the above-mentioned examples, the present application is not limited to the above-mentioned processes and steps, which means that the implementation of the present application does not necessarily depend on the above-mentioned processes and steps. It is to be apparent to those skilled in the art that any improvements made to the present application, equivalent replacements of raw materials

used in the present application, additions of adjuvant ingredients, selections of specific methods, etc., all fall within the protection scope and the disclosed scope of the present application.

## Claims

1. A crystal form A of proximod, wherein an X-ray powder diffraction pattern of the crystal form A has diffraction peaks at following diffraction angles (2θ): 3.2° ± 0.2°, 9.7° ± 0.2°, 12.9° ± 0.2°, 16.2° ± 0.2°, 19.5° ± 0.2°, 23.8° ± 0.2°, 26.5° ± 0.2°, and 26.8° ± 0.2°.

2. A preparation method for the crystal form A of proximod according to claim 1, comprising the following steps: adding a crude sample of proximod to a mixed solvent, keeping a pH of the system at 6-6.5, heating to dissolve the crude sample to a clear solution, adding the clear solution to methyl *tert*-butyl ether, stirring, precipitating a solid, separating the solid, and drying under reduced pressure to obtain the crystal form A.

3. The preparation method according to claim 2, wherein the mixed solvent is a mixed solvent of any one of methanol, ethanol or tetrahydrofuran and water.

4. The preparation method according to claim 2 or 3, wherein a volume ratio of methanol, ethanol or tetrahydrofuran to water in the mixed solvent is 9:1;

   preferably, the heating is heating to reflux;
   preferably, the drying under reduced pressure is performed at a temperature of 40 °C-50 °C.

5. A crystal form B of proximod, wherein an X-ray powder diffraction pattern of the crystal form B has diffraction peaks at following diffraction angles (2θ): 4.5° ± 0.2°, 9.0° ± 0.2°, 13.6° ± 0.2°, 18.1° ± 0.2°, 19.2° ± 0.2°, 21.8° ± 0.2°, 24.5° ± 0.2°, 27.5° ± 0.2°, and 27.8° ± 0.2°.

6. A preparation method for the crystal form B of proximod according to claim 5, comprising the following steps: adding the crystal form A of proximod according to claim 1 to a mixed solvent, adjusting a pH of the system to 1-2, heating to reflex until fully dissolved, cooling to 40 °C-50 °C, stirring, further cooling to room temperature, separating a solid, and drying under reduced pressure to obtain the crystal form.

7. The preparation method according to claim 6, wherein the adjusting the pH of the system is performed by using an acid, and the acid is concentrated hydrochloric acid;

   preferably, the mixed solvent comprises ethanol and water;
   preferably, a volume ratio of ethanol to water is 9:1;
   preferably, the drying under reduced pressure is performed at a temperature of 40 °C-55 °C;
   preferably, the seed of the crystal form B is added to the system after natural cooling to 40 °C-50 °C.

8. A pharmaceutical composition, comprising the crystal form A of proximod according to claim 1 and/or the crystal form B of proximod according to claim 5 and a pharmaceutically acceptable adjuvant.

9. The pharmaceutical composition according to claim 8, wherein the pharmaceutically acceptable adjuvant comprises microcrystalline cellulose.

10. The pharmaceutical composition according to claim 8 or 9, wherein the pharmaceutical composition comprises the crystal form A of proximod and a pharmaceutically acceptable adjuvant, and the pharmaceutically acceptable adjuvant comprises microcrystalline cellulose.

11. Use of the crystal form A of proximod according to claim 1, the crystal form B of proximod according to claim 5 or the pharmaceutical composition according to any one of claims 8 to 10 in the preparation of a drug having an immunomodulatory effect;

    preferably, the drug having the immunomodulatory effect is selected from drugs for treating immune disorders, hypoimmunity, immunosuppression, rejection reactions after organ transplantation or autoimmune diseases;
    preferably, the drug having the immunomodulatory effect is selected from drugs for treating rheumatoid arthritis.

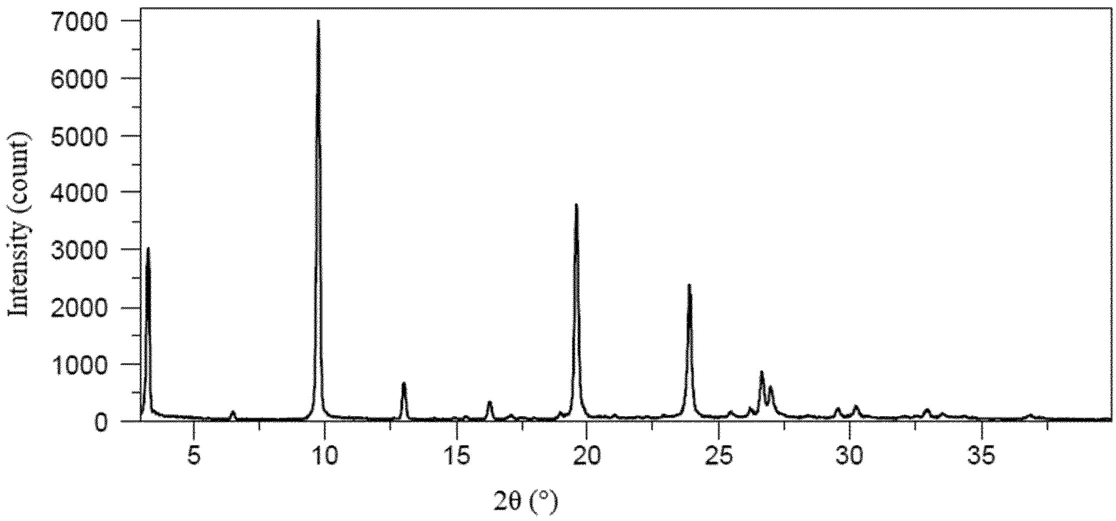
Intensity (count)
7000
6000
5000
4000
3000
2000
1000
0
5
10
15
20
25
30
35
2θ (°)

FIG. 1

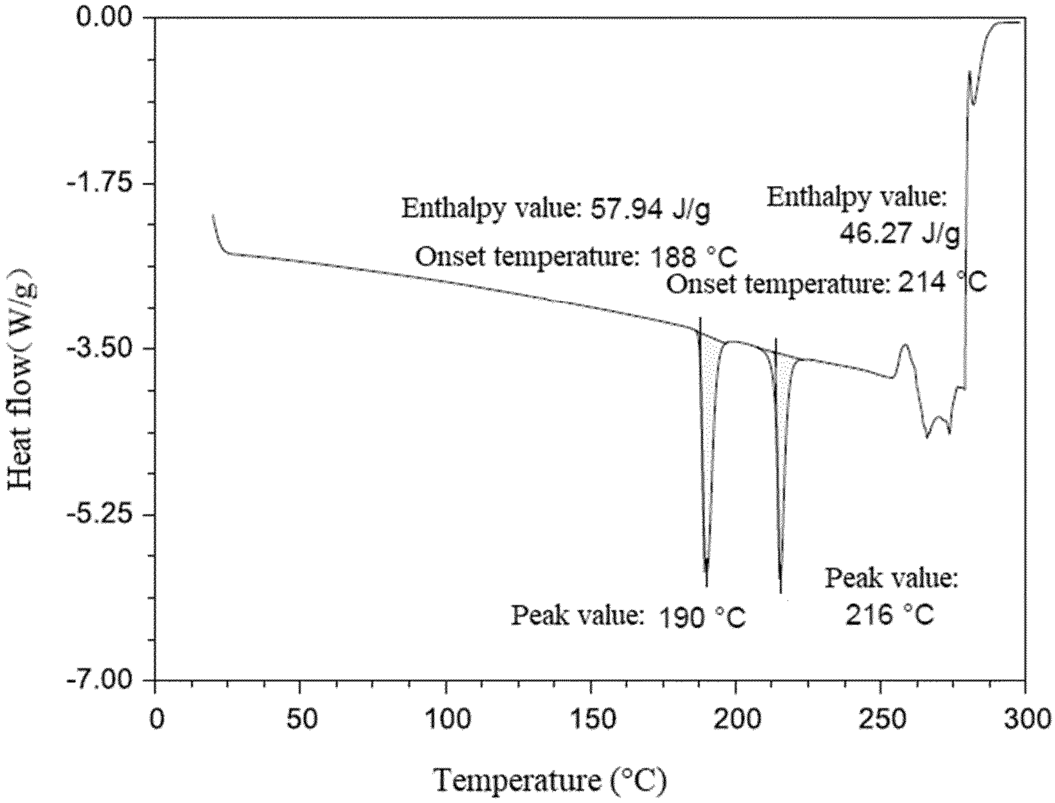
Heat flow( W/g )
0.00
-1.75
-3.50
-5.25
-7.00
Enthalpy value: 57.94 J/g
Onset temperature: 188 °C
Enthalpy value:
46.27 J/g
Onset temperature: 214 °C
Peak value: 190 °C
Peak value:
216 °C
0
50
100
150
200
250
300
Temperature (°C)

FIG. 2

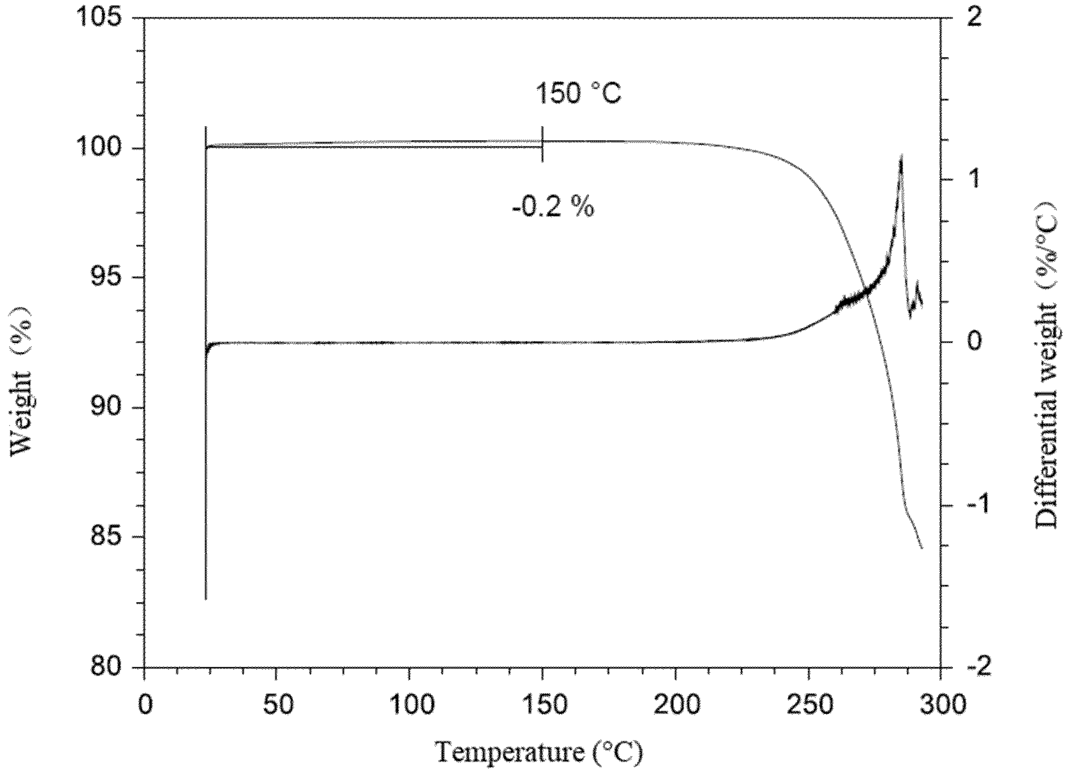
150 °C
-0.2 %
Weight（%）
Differential weight（%/°C）
Temperature (°C)
105
100
95
90
85
80
2
1
0
-1
-2
0
50
100
150
200
250
300

FIG. 3

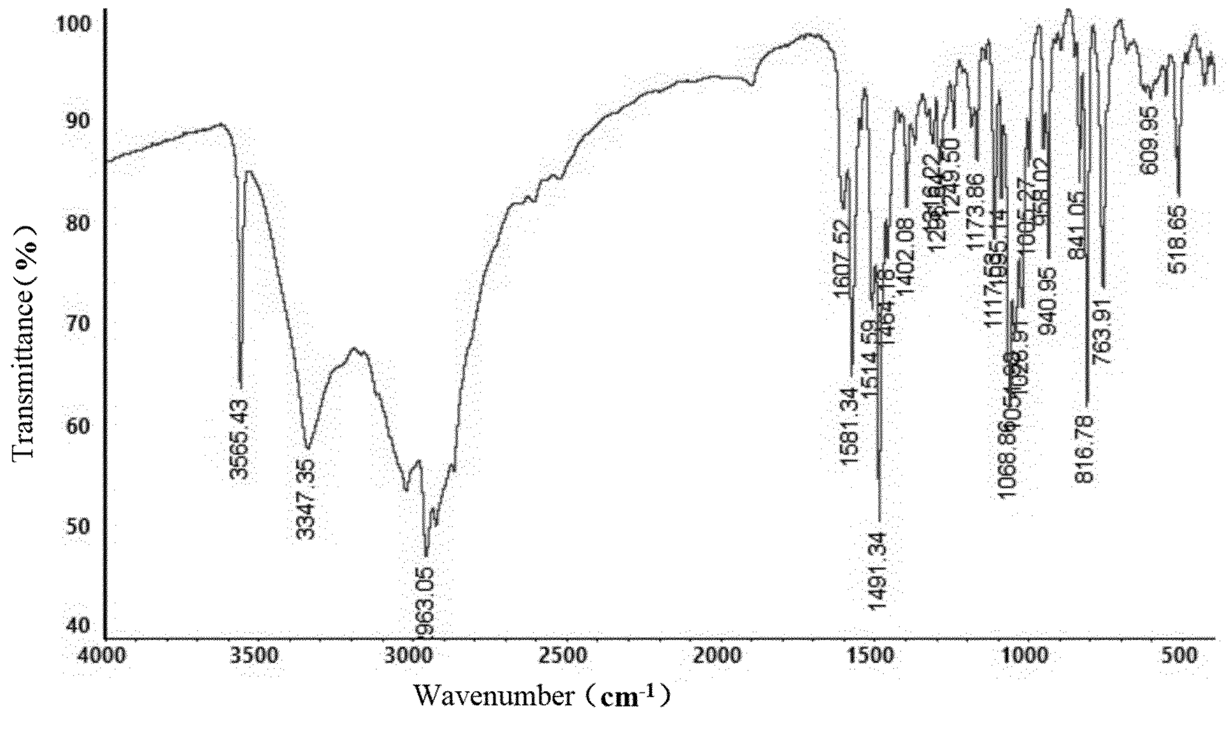
Transmittance (%)
Wavenumber (cm-1)
100
90
80
70
60
50
40
4000
3500
3000
2500
2000
1500
1000
500
3565.43
3347.35
963.05
1607.52
1581.34
1514.59
1491.34
1464.16
1402.08
1249.50
1173.86
1005.27
958.02
940.95
841.05
816.78
763.91
609.95
518.65
1068.86

FIG. 4

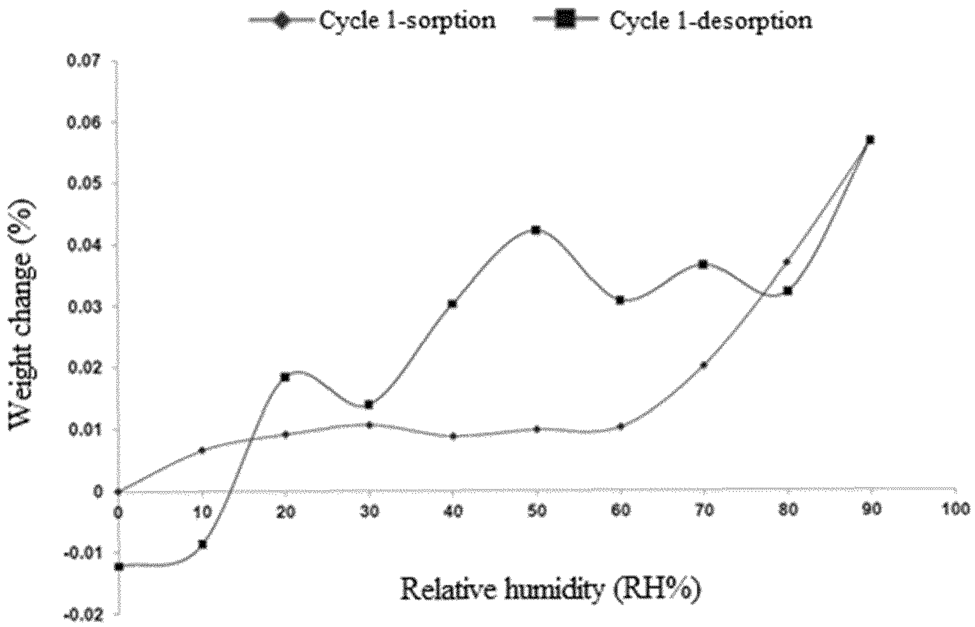
Cycle 1-sorption
Cycle 1-desorption
Weight change (%)
0.07
0.06
0.05
0.04
0.03
0.02
0.01
0
-0.01
-0.02
0
10
20
30
40
50
60
70
80
90
100
Relative humidity (RH%)

FIG. 5

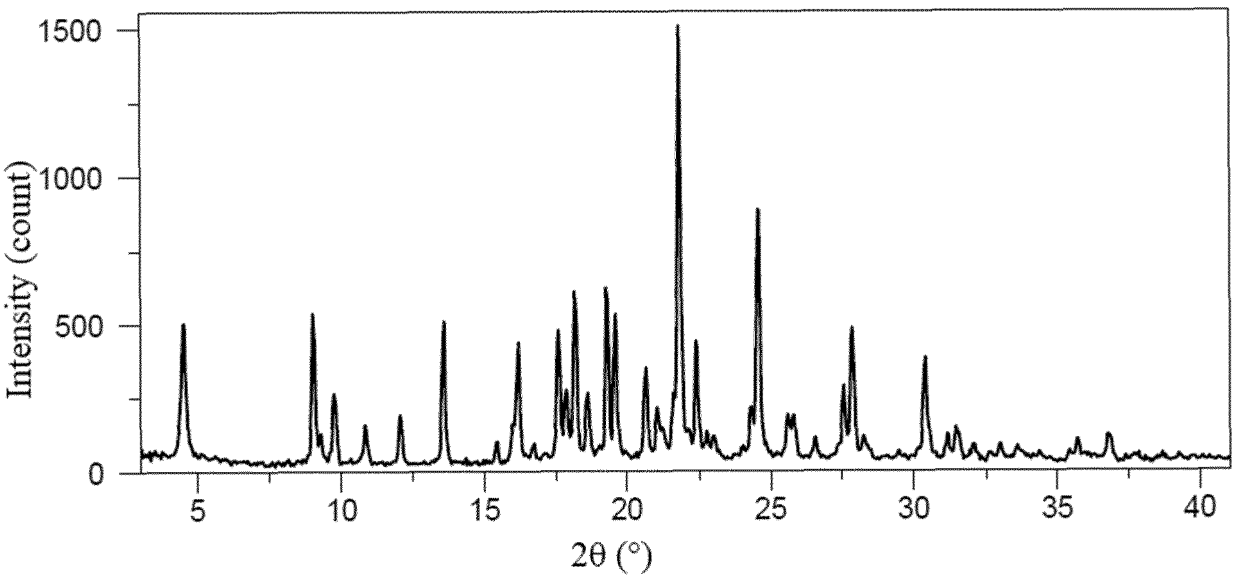
Intensity (count)
1500
1000
500
0
5
10
15
20
25
30
35
40
2θ (°)

FIG. 6

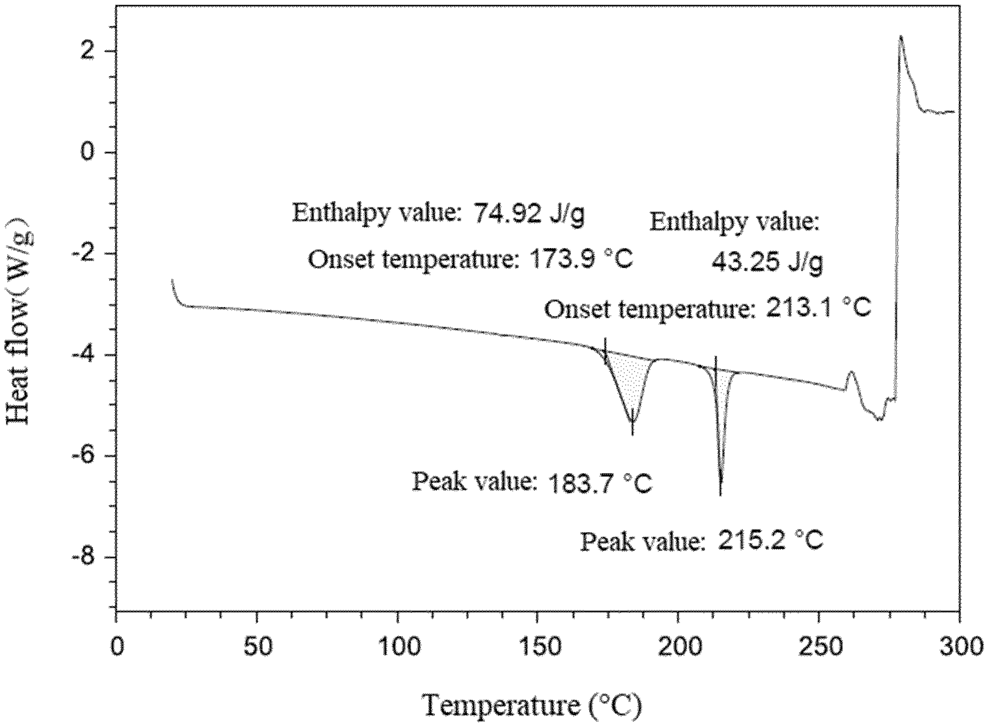
Enthalpy value: 74.92 J/g
Onset temperature: 173.9 °C
Enthalpy value: 43.25 J/g
Onset temperature: 213.1 °C
Peak value: 183.7 °C
Peak value: 215.2 °C
Heat flow (W/g)
Temperature (°C)
2
0
-2
-4
-6
-8
0
50
100
150
200
250
300

FIG. 7

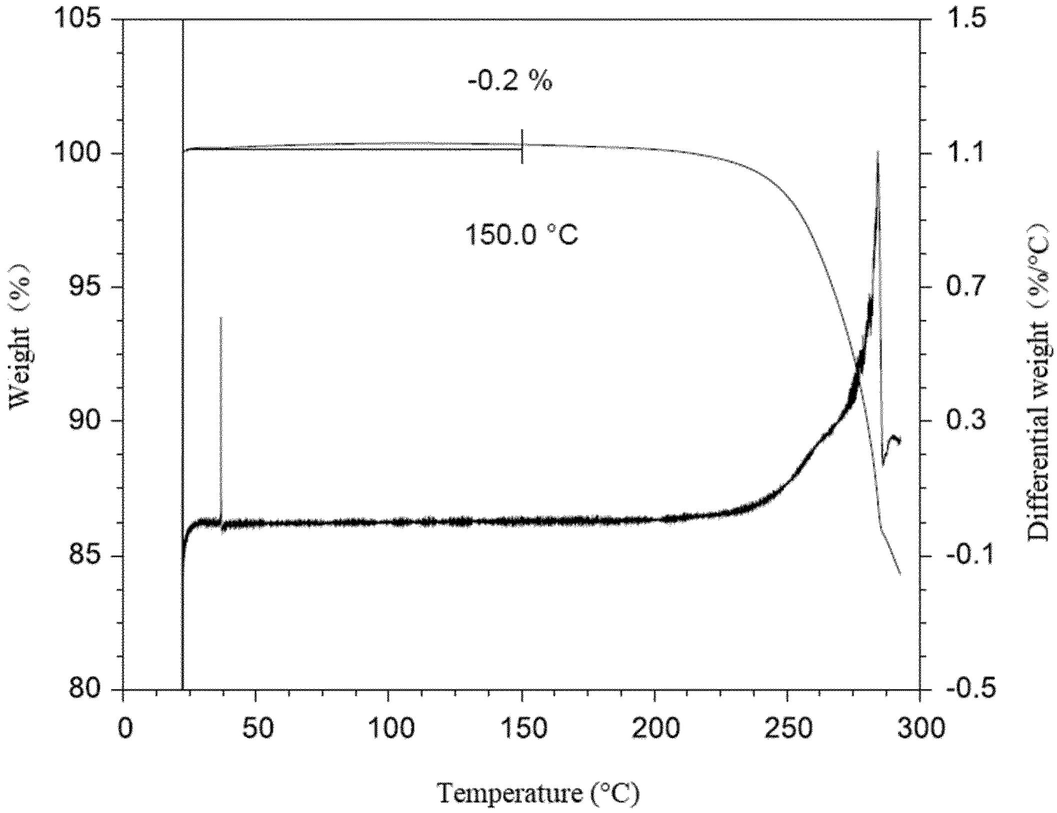
-0.2 %
150.0 °C
Weight (%)
Differential weight (%/°C)
Temperature (°C)
105
100
95
90
85
80
1.5
1.1
0.7
0.3
-0.1
-0.5
0
50
100
150
200
250
300

FIG. 8

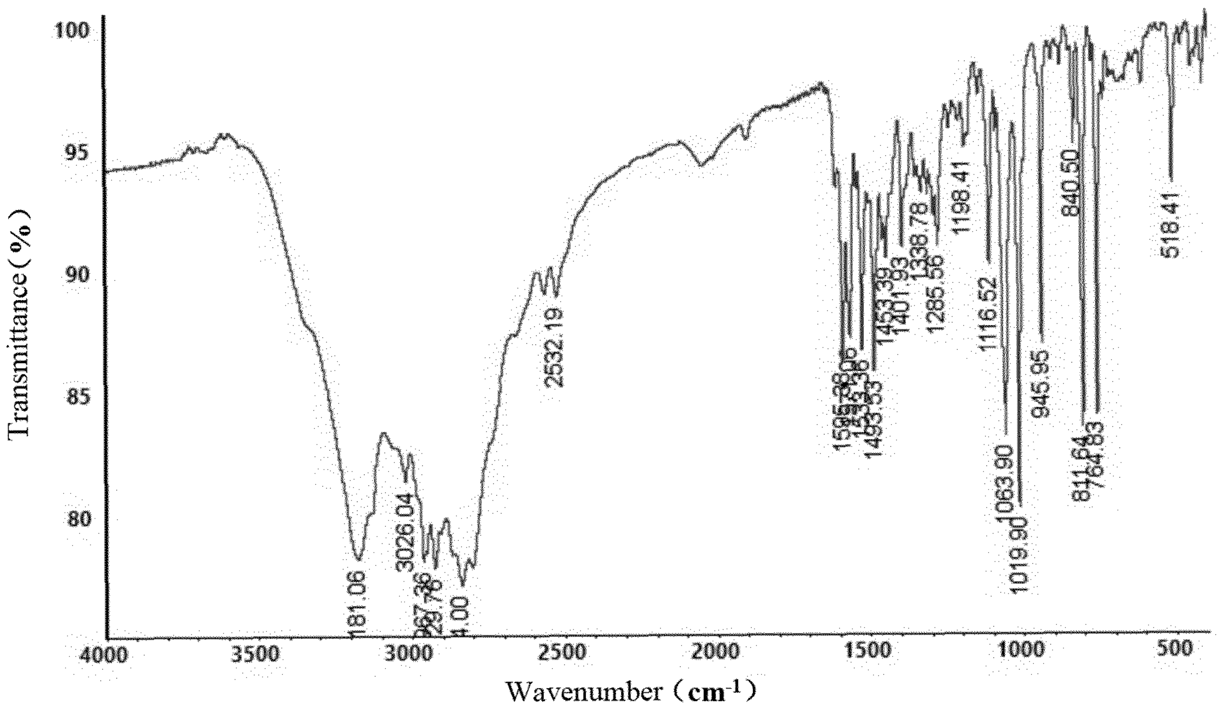
Transmittance (%)
Wavenumber (cm-1)
100
95
90
85
80
4000
3500
3000
2500
2000
1500
1000
500
3026.04
2532.19
1453.39
1401.93
1338.78
1285.56
1198.41
1116.52
1063.90
1019.90
945.95
840.50
518.41
1493.53
811.64
764.83

FIG. 9

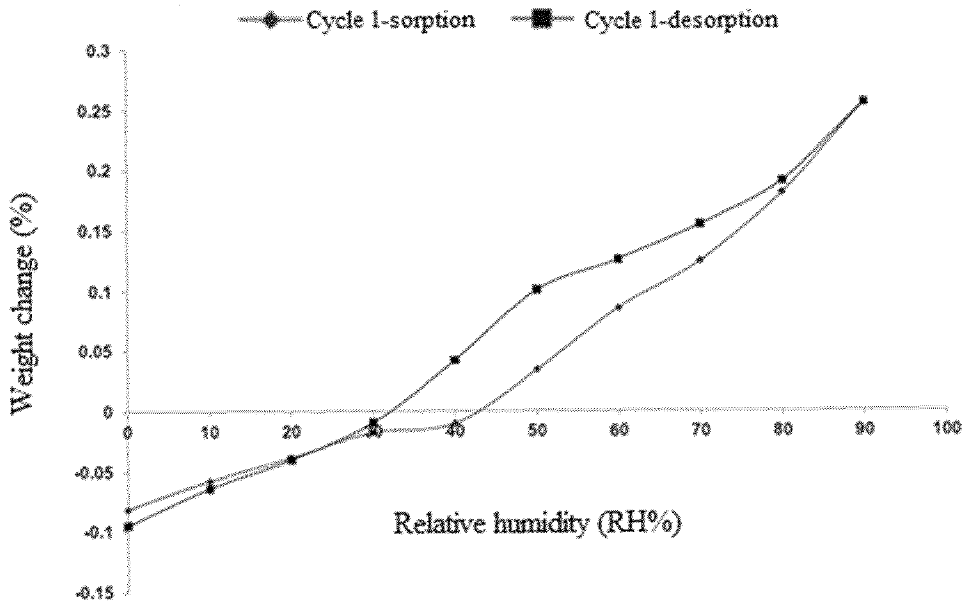
Cycle 1-sorption
Cycle 1-desorption
Weight change (%)
Relative humidity (RH%)
0.3
0.25
0.2
0.15
0.1
0.05
0
-0.05
-0.1
-0.15
0
10
20
30
40
50
60
70
80
90
100

FIG. 10

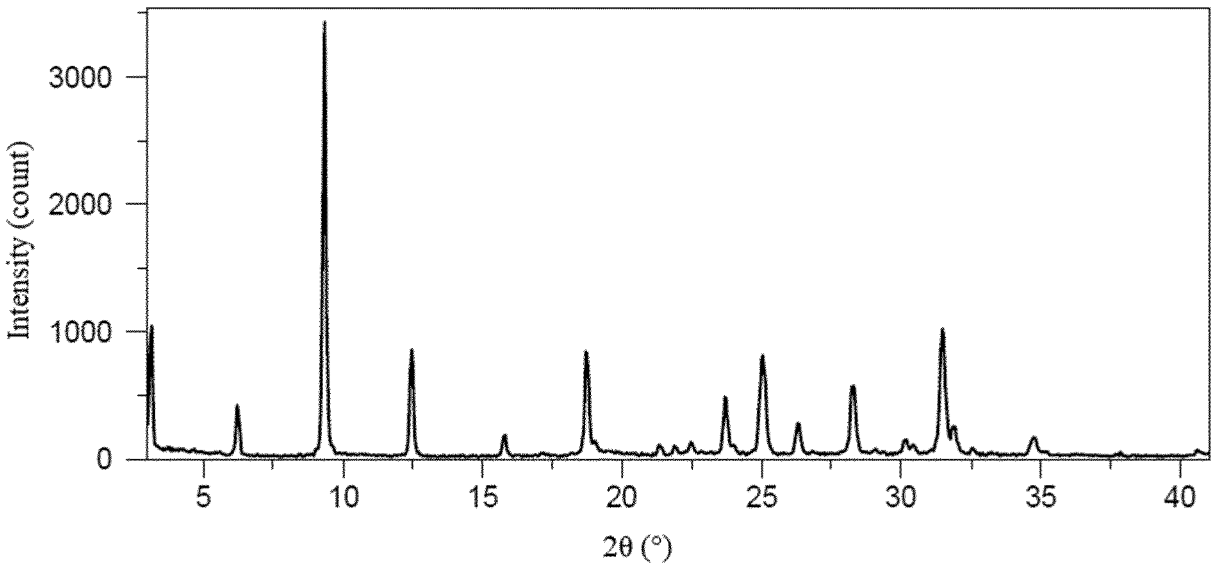
Intensity (count)
3000
2000
1000
0
5
10
15
20
25
30
35
40
2θ (°)

FIG. 11

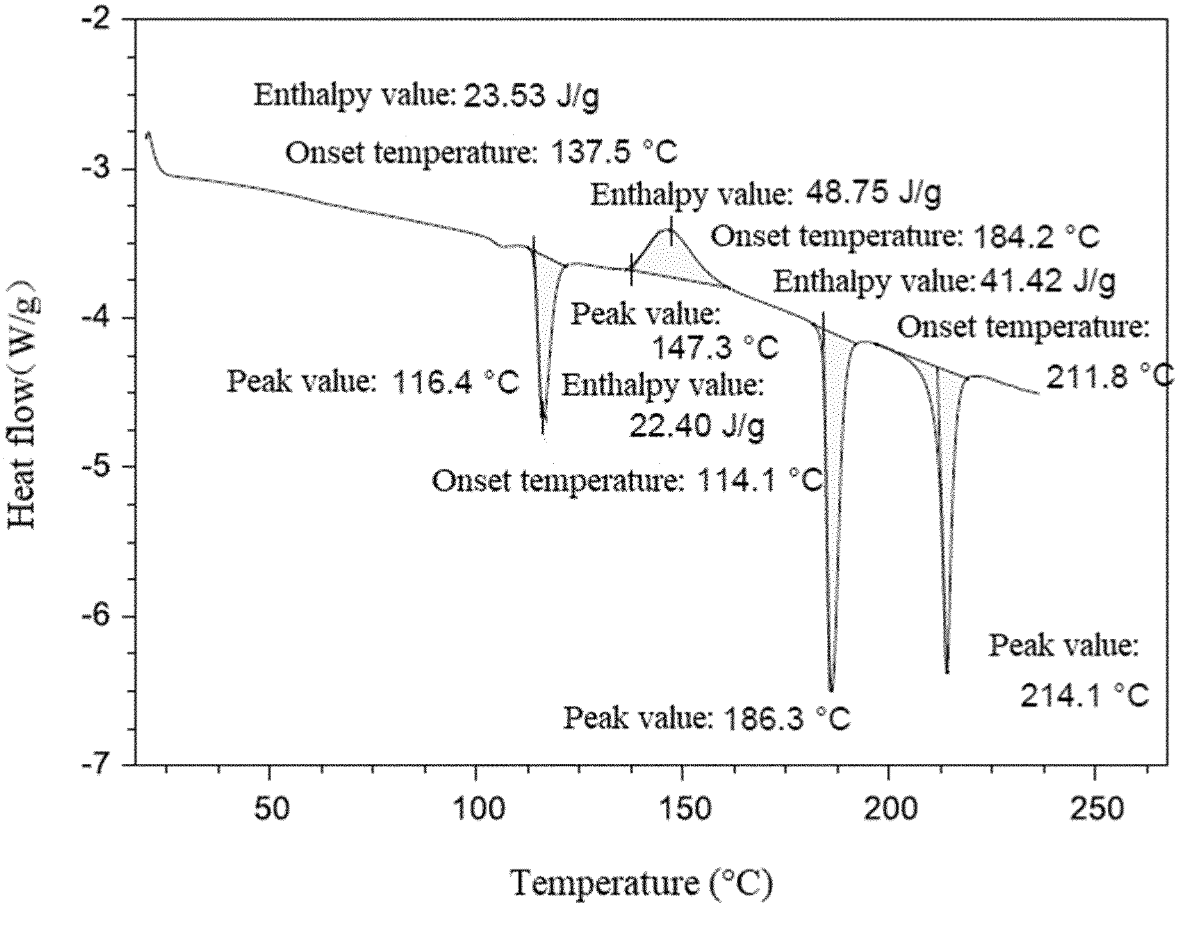
Heat flow( W/g)
Temperature (°C)
-2
-3
-4
-5
-6
-7
50
100
150
200
250
Enthalpy value: 23.53 J/g
Onset temperature: 137.5 °C
Enthalpy value: 48.75 J/g
Onset temperature: 184.2 °C
Enthalpy value: 41.42 J/g
Onset temperature:
211.8 °C
Peak value:
147.3 °C
Peak value: 116.4 °C
Enthalpy value:
22.40 J/g
Onset temperature: 114.1 °C
Peak value:
214.1 °C
Peak value: 186.3 °C

FIG. 12

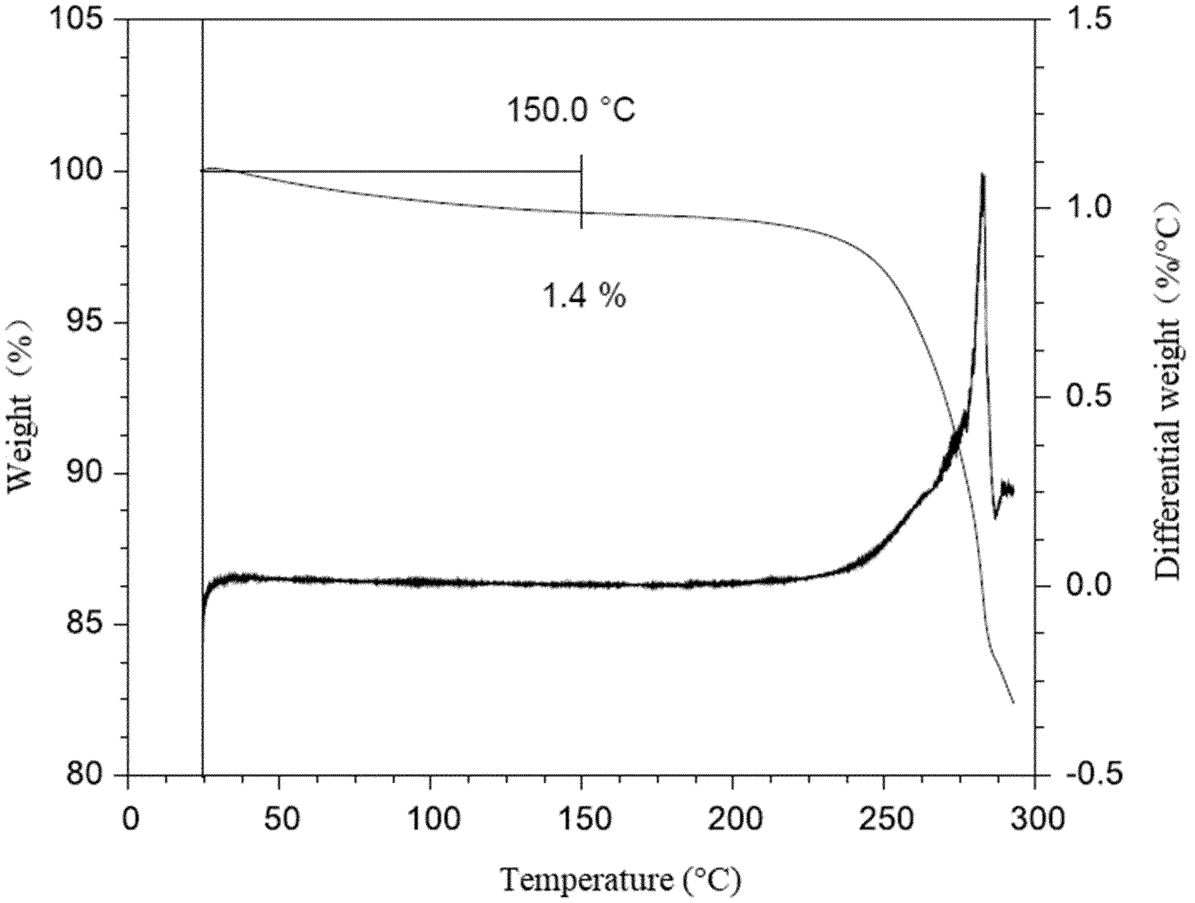
150.0 °C
1.4 %
Weight（%）
Differential weight（%/°C）
Temperature (°C)
105
100
95
90
85
80
0
50
100
150
200
250
300
1.5
1.0
0.5
0.0
-0.5

FIG. 13

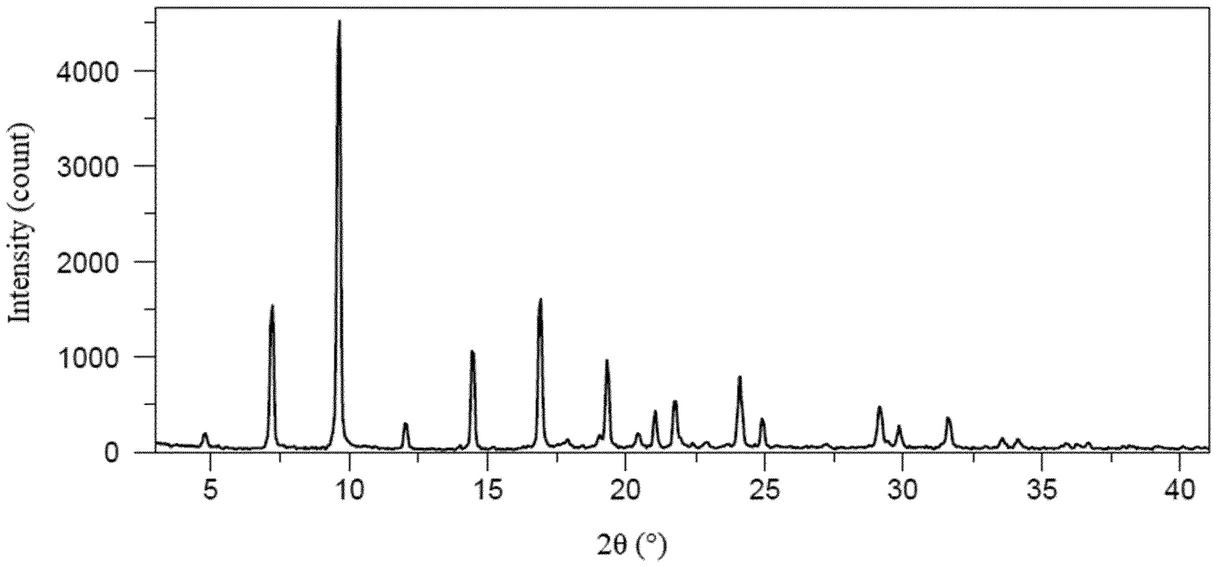
Intensity (count)
2θ (°)
0
1000
2000
3000
4000
5
10
15
20
25
30
35
40

FIG. 14

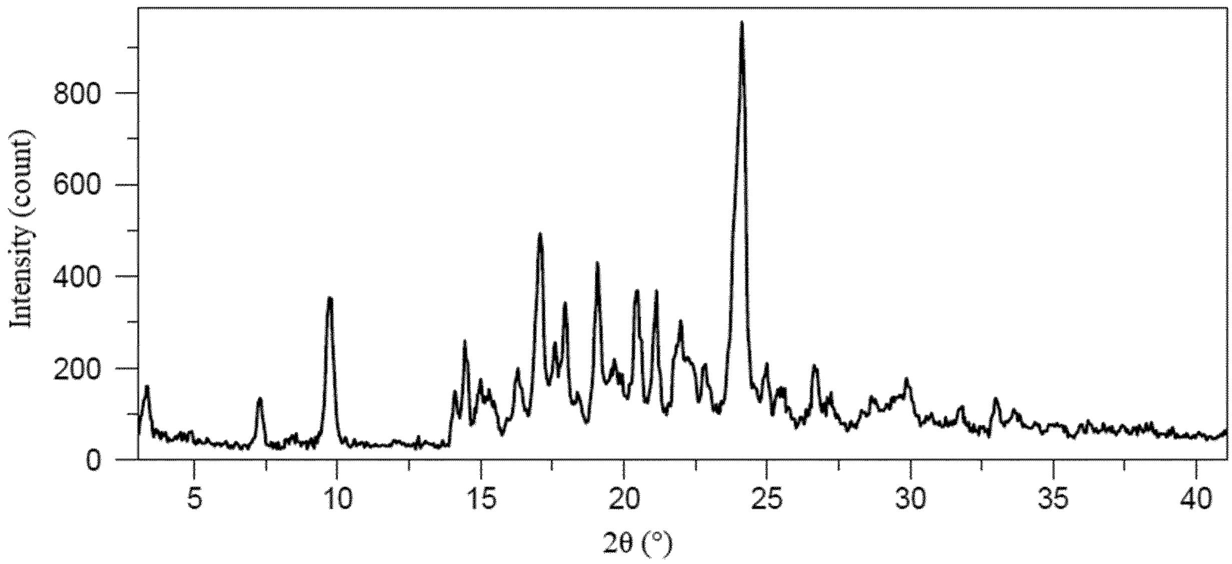
Intensity (count)
800
600
400
200
0
5
10
15
20
25
30
35
40
2θ (°)

FIG. 15

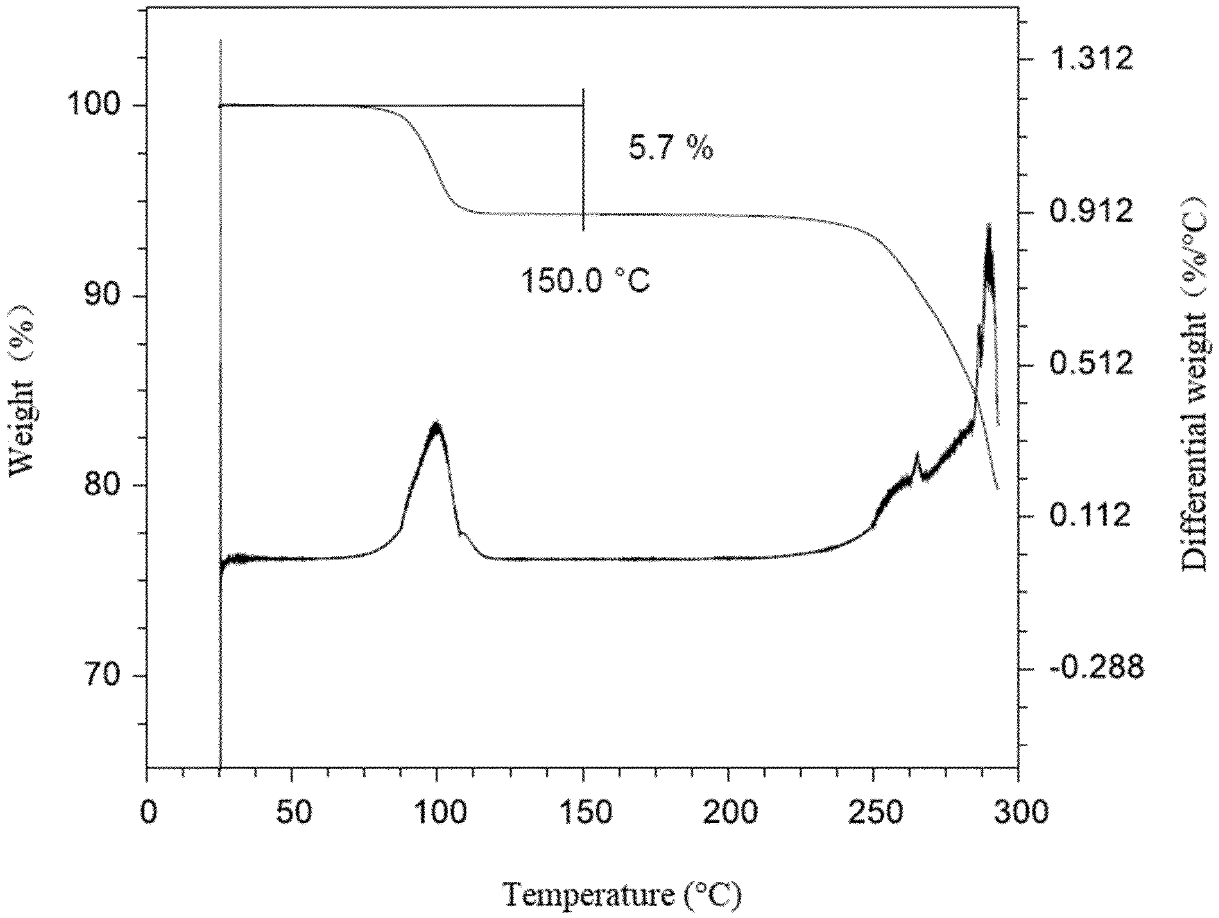
100
90
80
70
Weight（%）
1.312
0.912
0.512
0.112
-0.288
Differential weight（%/°C）
5.7 %
150.0 °C
0
50
100
150
200
250
300
Temperature (°C)

FIG. 16

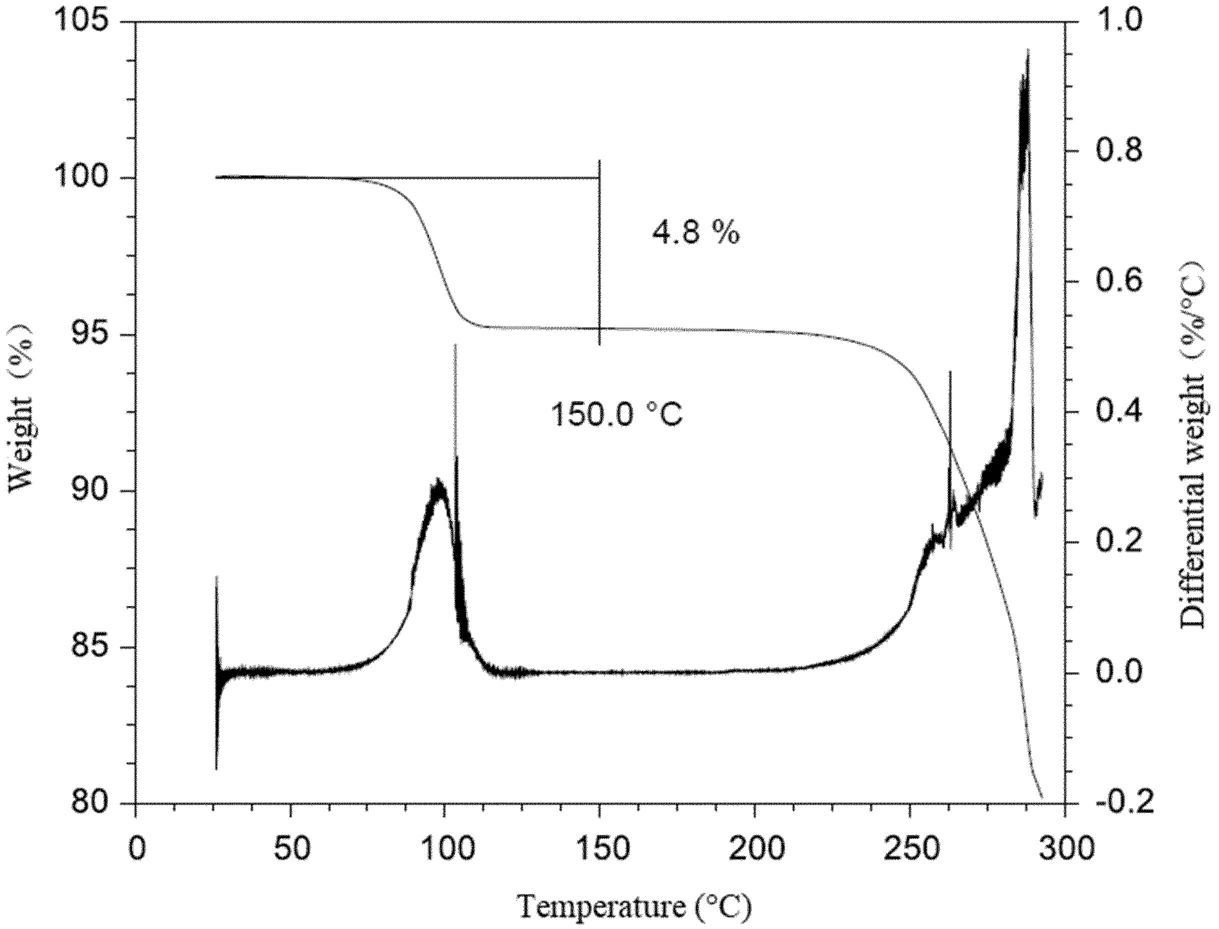
4.8 %
150.0 °C
Weight (%)
Differential weight (%/°C)
Temperature (°C)

FIG. 17

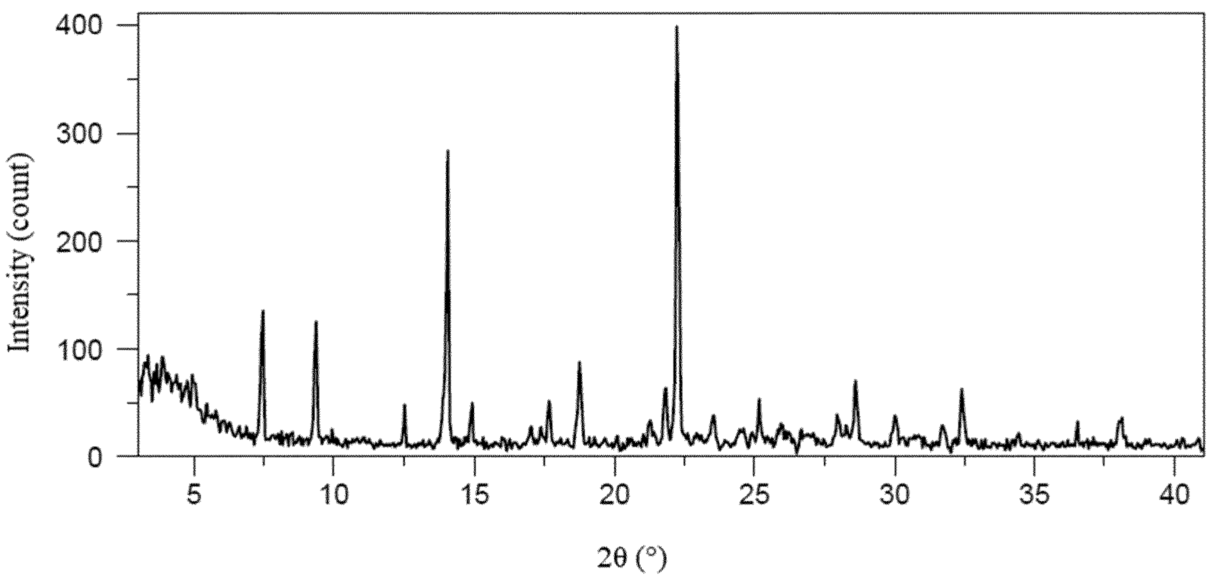
Intensity (count)
2θ (°)

FIG. 18

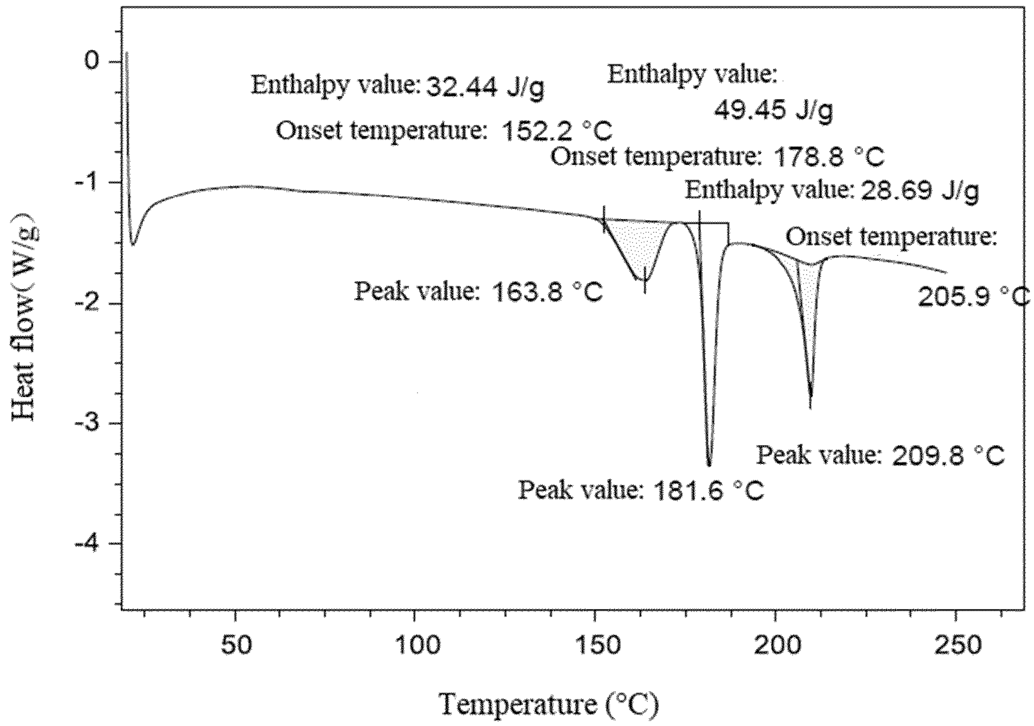
Enthalpy value: 32.44 J/g
Onset temperature: 152.2 °C
Enthalpy value: 49.45 J/g
Onset temperature: 178.8 °C
Enthalpy value: 28.69 J/g
Onset temperature: 205.9 °C
Peak value: 163.8 °C
Peak value: 181.6 °C
Peak value: 209.8 °C
Heat flow (W/g)
Temperature (°C)
0
-1
-2
-3
-4
50
100
150
200
250

FIG. 19

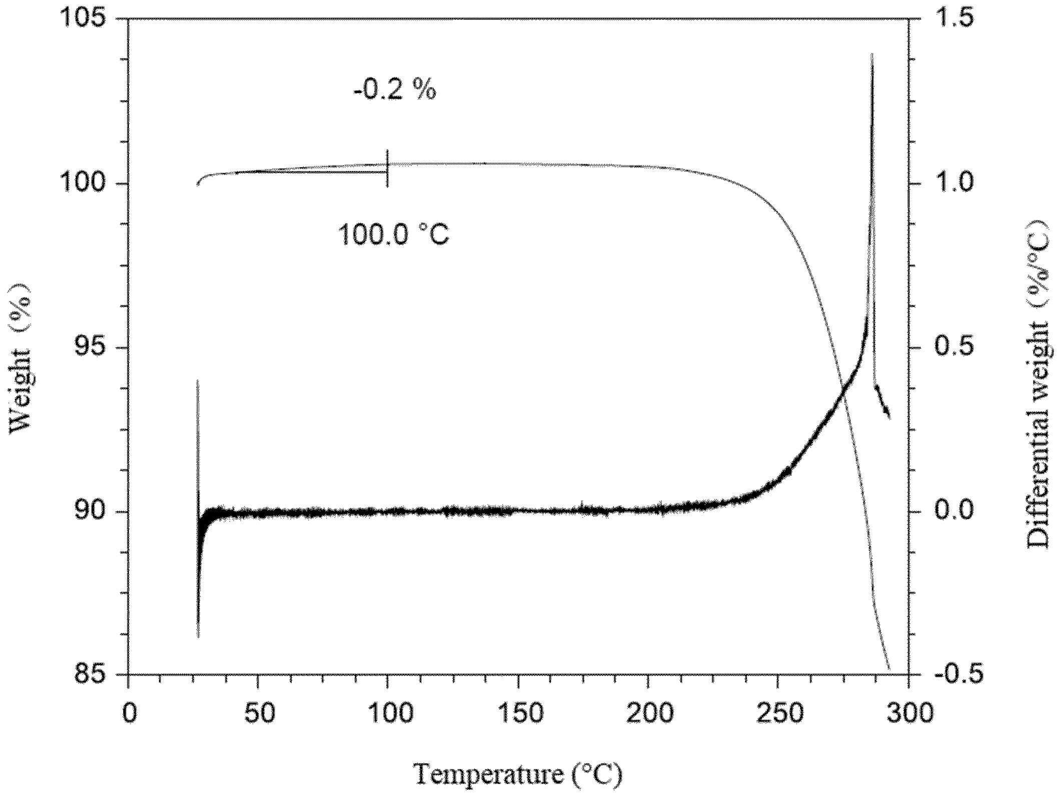
-0.2 %
100.0 °C
Weight (%)
Differential weight (%/°C)
Temperature (°C)
105
100
95
90
85
1.5
1.0
0.5
0.0
-0.5
0
50
100
150
200
250
300

FIG. 20

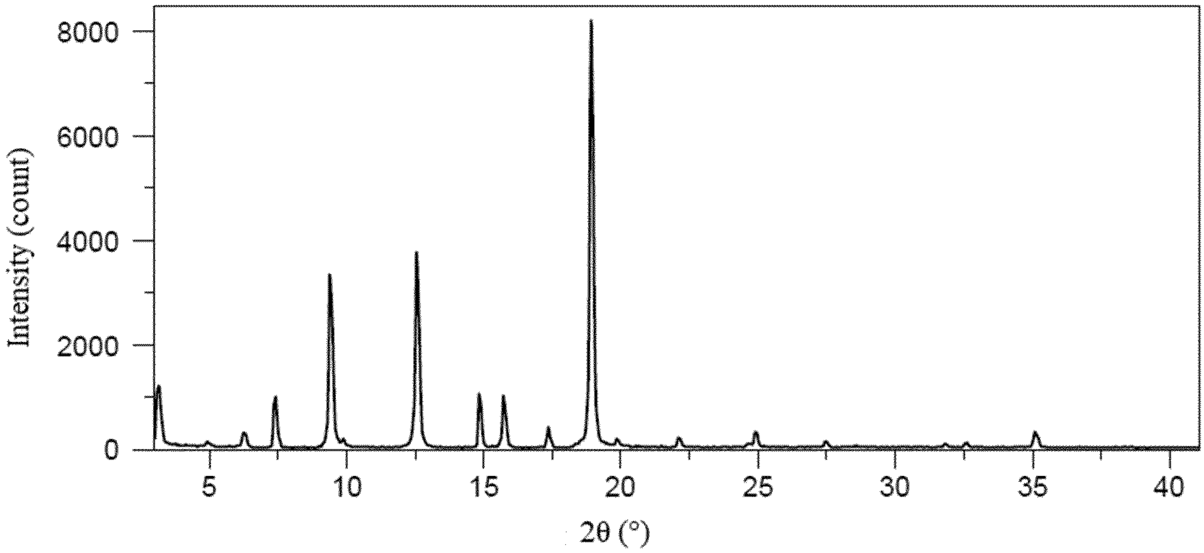


FIG. 21

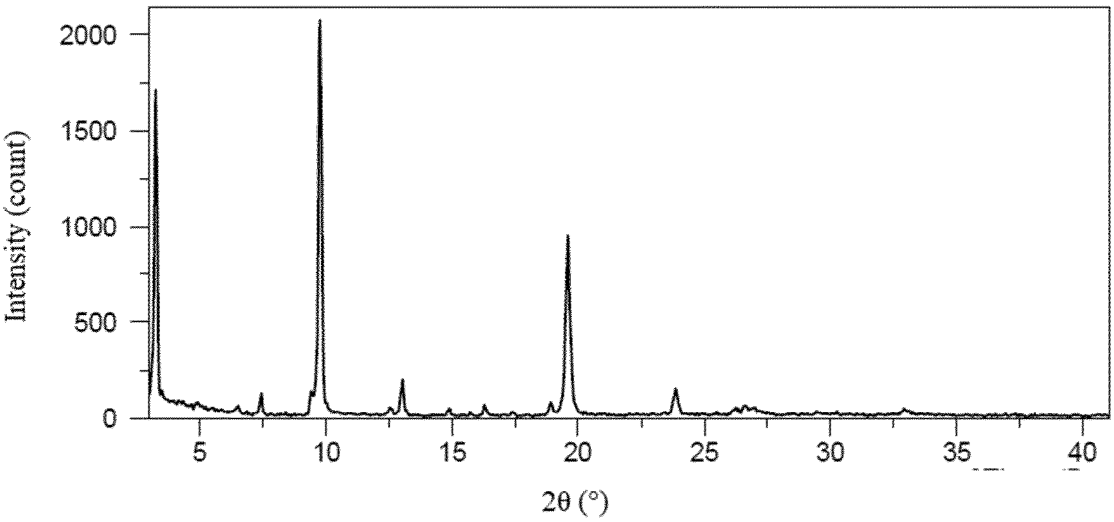


FIG. 22

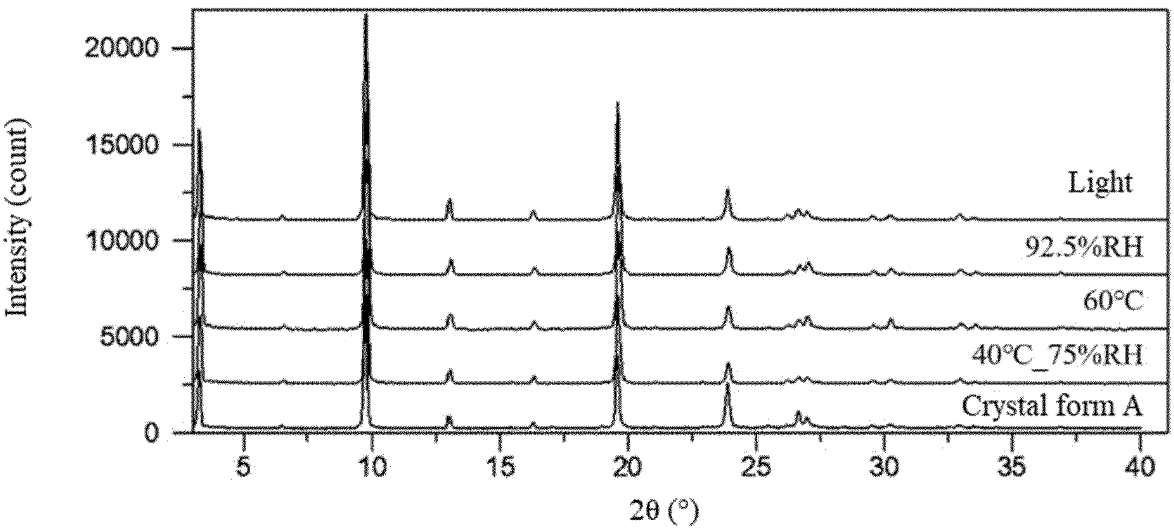


FIG. 23

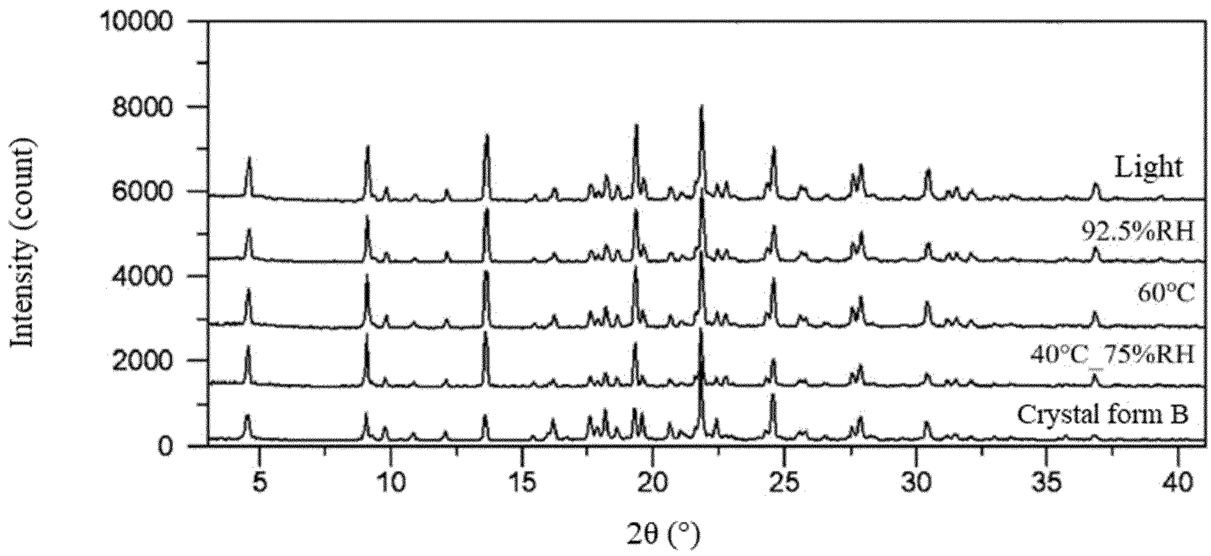


FIG. 24

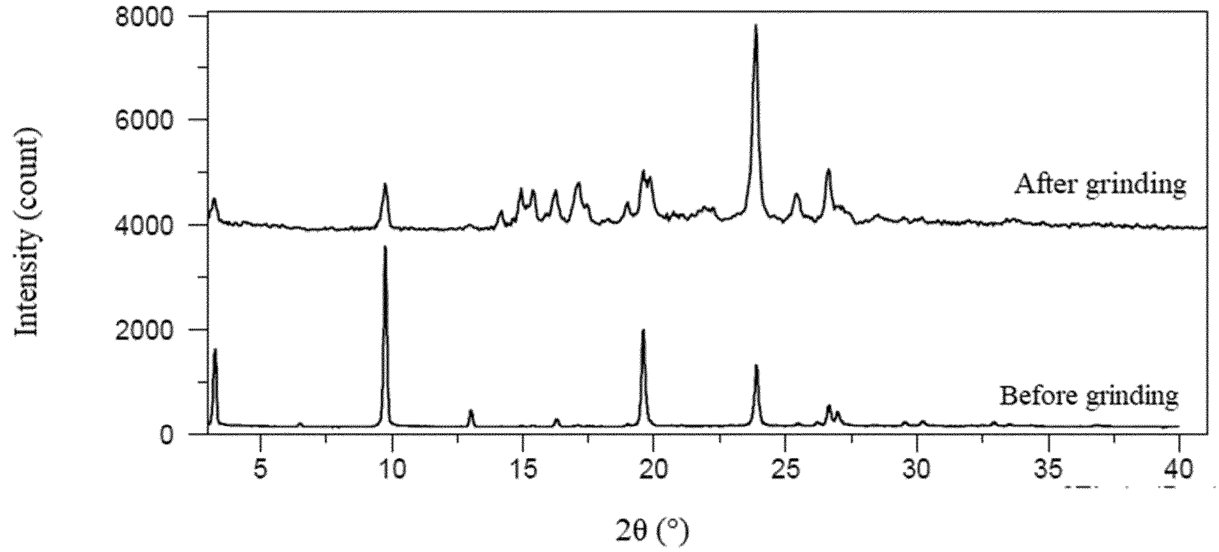


FIG. 25

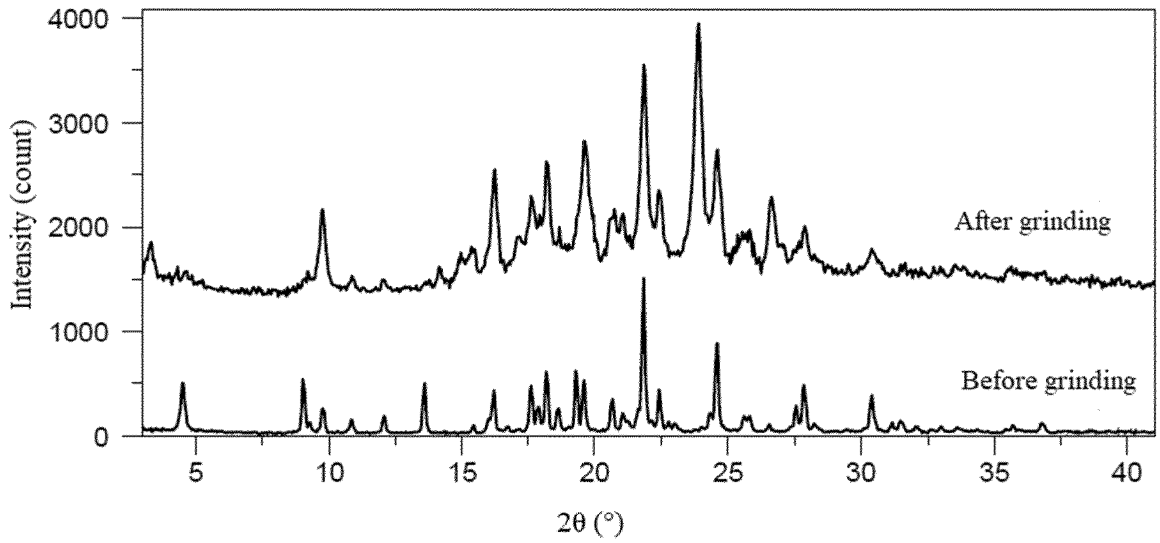


FIG. 26

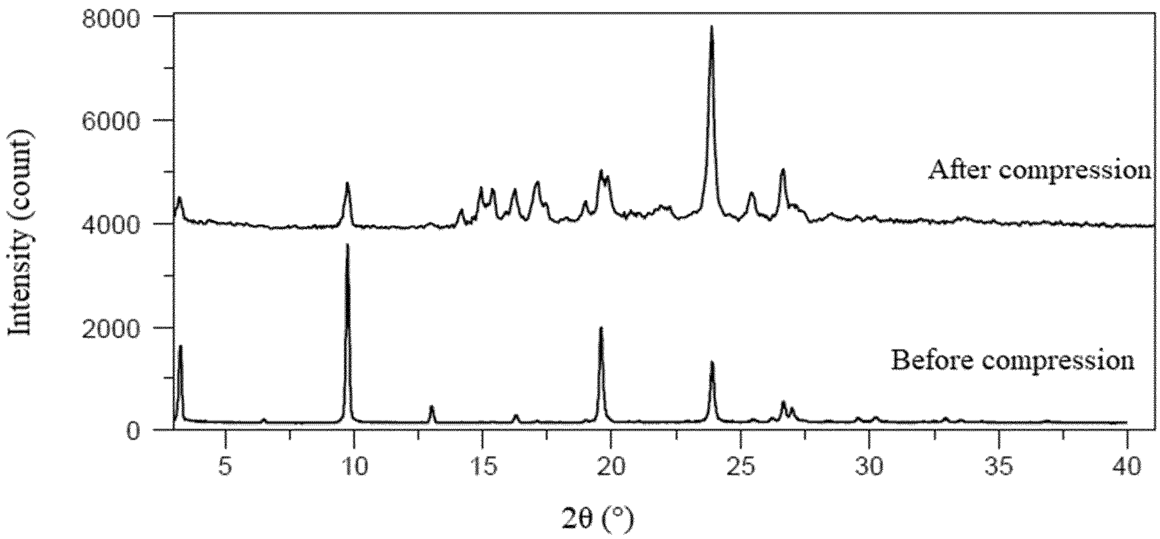
8000
6000
4000
2000
0
Intensity (count)
5
10
15
20
25
30
35
40
2θ (°)
After compression
Before compression

FIG. 27

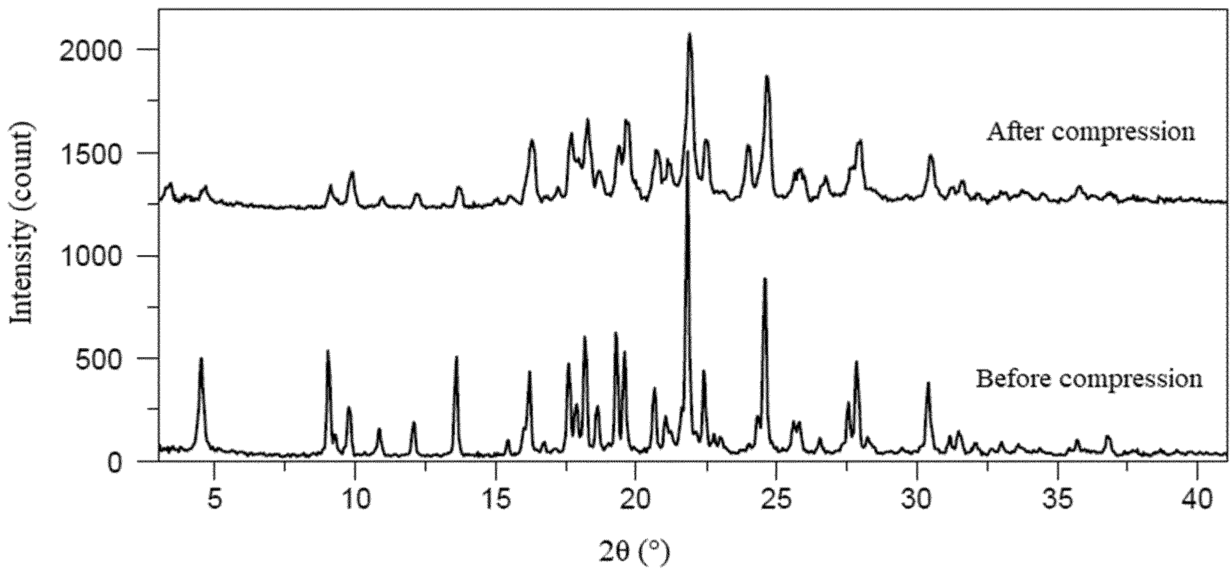
2000
1500
1000
500
0
Intensity (count)
5
10
15
20
25
30
35
40
2θ (°)
After compression
Before compression

FIG. 28

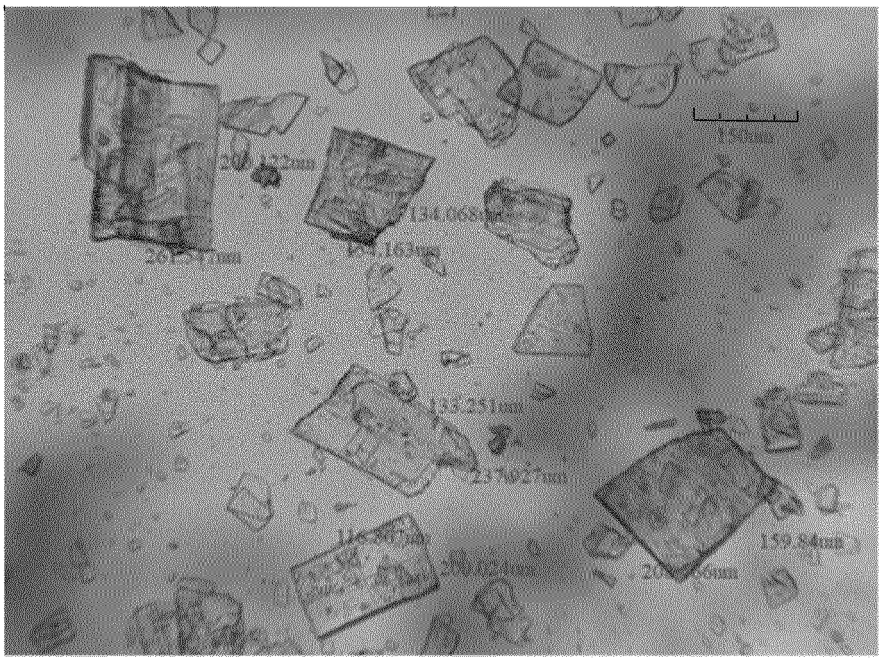
150um
133.251um
237.927um
200.024um
159.84um

FIG. 29

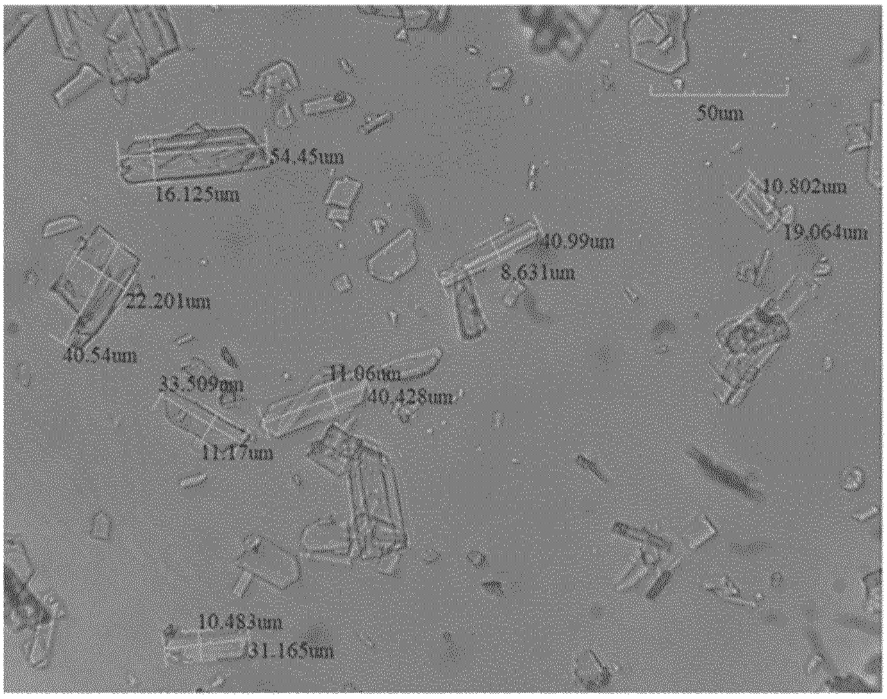
50um
54.45um
16.125um
10.802um
19.064um
40.99um
8.631um
22.201um
40.54um
33.509um
11.06um
40.428um
11.17um
10.483um
31.165um

FIG. 30

**INTERNATIONAL SEARCH REPORT**

International application No.
**PCT/CN2023/137500**

**A. CLASSIFICATION OF SUBJECT MATTER**

C07D263/32(2006.01)i; A61P37/02(2006.01)i; A61P37/04(2006.01)i; A61P37/06(2006.01)i; A61P19/02(2006.01)i; A61K31/421(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNTXT, WPABS, WPABSC, STN on the web: 普赛莫得, SYL930, S1P1, 1418093-75-0, 1417987-55-3

**C. DOCUMENTS CONSIDERED TO BE RELEVANT**

| Category* | Citation of document, with indication, where appropriate, of the relevant passages | Relevant to claim No. |
|---|---|---|
| X | CN 111087357 A (INSTITUTE OF MATERIA MEDICA, CHINESE ACADEMY OF MEDICAL SCIENCES) 01 May 2020 (2020-05-01) description, paragraph [0002], and embodiment 5 | 1-11 |
| X | CN 111087358 A (INSTITUTE OF MATERIA MEDICA, CHINESE ACADEMY OF MEDICAL SCIENCES) 01 May 2020 (2020-05-01) description, paragraph [0002], and embodiment 5 | 1-11 |
| A | CN 102863345 A (INSTITUTE OF MATERIA MEDICA, CHINESE ACADEMY OF MEDICAL SCIENCES) 09 January 2013 (2013-01-09) second-to-last compound of claim 15, and claims 16-18 | 1-11 |
| A | XIAO, Qiong et al. "Synthesis, identification, and biological activity of metabolites of two novel selective S1P1 agonists" *Bioorg. Med. Chem.,* Vol. 24, 31 March 2016 (2016-03-31), 2273-2279 | 1-11 |
| PX | CN 115974803 A (JIANKUAN (SUZHOU) BIOTECHNOLOGY CO., LTD. et al.) 18 April 2023 (2023-04-18) claims 1-10 | 1-11 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 January 2024** | **01 February 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/137500**

| Patent document cited in search report | Publication date (day/month/year) | Patent family member(s) | Publication date (day/month/year) |
|---|---|---|---|
| CN 111087357 A | 01 May 2020 | None | |
| CN 111087358 A | 01 May 2020 | None | |
| CN 102863345 A | 09 January 2013 | WO 2013004190 A1 | 10 January 2013 |
| | | EP 2786982 A1 | 08 October 2014 |
| | | EP 2786982 A4 | 15 April 2015 |
| | | JP 2014523887 A | 18 September 2014 |
| | | US 2014323501 A1 | 30 October 2014 |
| | | KR 20140048239 A | 23 April 2014 |
| | | EA 201490221 A1 | 30 December 2014 |
| CN 115974803 A | 18 April 2023 | None | |

Form PCT/ISA/210 (patent family annex) (July 2022)